# EUROPÄISCHE PATENTSCHRIFT

(11) **EP 2 875 019 B1**
(45) Veröffentlichungstag und Bekanntmachung des Hinweises auf die Patenterteilung: **29.03.2017**
(21) Anmeldenummer: 13732363.0
(22) Anmeldetag: 25.06.2013
(51) Int. Cl.: C07D 403/10

(54) **MATERIALIEN FÜR ORGANISCHE ELEKTROLUMINESZENZVORRICHTUNGEN**
MATERIALS FOR ORGANIC ELECTROLUMINESCENCE DEVICES
MATÉRIAUX POUR DISPOSITIFS ÉLECTROLUMINESCENTS ORGANIQUES

(30) Priorität: 23.07.2012 EP 12005368
(43) Veröffentlichungstag der Anmeldung: 27.05.2015
(73) Patentinhaber: Merck Patent GmbH, 64293 Darmstadt (DE)
(72) Erfinder: PARHAM, Amir Hossain, 65929 Frankfurt am Main (DE); EBERLE, Thomas, 76829 Landau (DE); JATSCH, Anja, 60489 Frankfurt am Main (DE); PFLUMM, Christof, 64291 Darmstadt (DE); KROEBER, Jonas Valentin, 60311 Frankfurt am Main (DE)
(86) Internationale Anmeldenummer: PCT/EP2013/001861
(87) Internationale Veröffentlichungsnummer: WO 2014/015931

(56) Entgegenhaltungen:
- WO-A2-2010/131930
- WO-A2-2011/057706
- WO-A2-2012/074210

## Beschreibung

Die vorliegende Erfindung beschreibt Carbazol-Derivate, insbesondere zur Verwendung als Triplettmatrixmaterialien in organischen Elektrolumineszenzvorrichtungen. Die Erfindung betrifft ferner ein Verfahren zur Herstellung der erfindungsgemäßen Verbindungen sowie elektronische Vorrichtungen enthaltend diese Verbindungen.

Der Aufbau organischer Elektrolumineszenzvorrichtungen (OLEDs), in denen organische Halbleiter als funktionelle Materialien eingesetzt werden, ist beispielsweise in US 4539507, US 5151629, EP 0676461 und WO 98/27136 beschrieben. Als emittierende Materialien werden häufig metallorganische Komplexe eingesetzt, die Phosphoreszenz statt Fluoreszenz zeigen. Aus quantenmechanischen Gründen ist unter Verwendung metallorganischer Verbindungen als Phosphoreszenzemitter eine bis zu vierfache Energie- und Leistungseffizienz möglich. Generell gibt es bei OLEDs, insbesondere auch bei OLEDs, die Triplettemission (Phosphoreszenz) zeigen, immer noch Verbesserungsbedarf, beispielsweise im Hinblick auf Effizienz, Betriebsspannung und Lebensdauer.

Die Eigenschaften phosphoreszierender OLEDs werden nicht nur von den eingesetzten Triplettemittern bestimmt. Hier sind insbesondere auch die anderen verwendeten Materialien wie zum Beispiel Matrixmaterialien von besonderer Bedeutung. Verbesserungen dieser Materialien können somit auch zu deutlichen Verbesserungen der OLED-Eigenschaften führen.

Gemäß dem Stand der Technik werden unter anderem Indolocarbazolderivate (z. B. gemäß WO 2007/063754 oder WO 2008/056746) oder Indenocarbazolderivate (z. B. gemäß WO 2010/136109 oder WO 2011/000455), insbesondere solche, die mit elektronenarmen Heteroaromaten wie Triazin substituiert sind, als Matrixmaterialien für phosphoreszierende Emitter verwendet. Weiterhin werden beispielsweise Bisdibenzofuranderivate (z. B. gemäß EP 2301926) als Matrixmaterialien für phosphoreszierende Emitter verwendet. Allerdings besteht bei Verwendung dieser Matrixmaterialien noch Verbesserungsbedarf, insbesondere in Bezug auf die Effizienz, die Lebensdauer und die Betriebsspannung der Vorrichtung.

Aufgabe der vorliegenden Erfindung ist die Bereitstellung von Verbindungen, welche sich für den Einsatz in einer fluoreszierenden oder insbesondere in einer phosphoreszierenden OLED eignen, insbesondere als Matrixmaterial. Insbesondere ist es die Aufgabe der vorliegenden Erfindung, Matrixmaterialien bereitzustellen, welche sich auch für grün und gegebenenfalls auch für blau phosphoreszierende OLEDs eignen, und die zu guter Effizienz, hoher Lebensdauer und geringer Betriebsspannung führen. Gerade auch die Eigenschaften der Matrixmaterialien haben einen wesentlichen Einfluss auf die Lebensdauer und die Effizienz der organischen Elektrolumineszenzvorrichtung.

Aus der WO 2011/057706 sind Carbazolderivate bekannt, welche mit zwei Triphenyltriazingruppen substituiert sind. Hier sind noch weitere Verbesserungen, insbesondere in Bezug auf das Triplettniveau sowie auf die Sublimationsstabilität wünschenswert.

Aus der WO 2011/055914 ist ein Carbazolderivat bekannt, welche mit einer Triphenyltriazingruppe substituiert ist, wobei eine der Phenylgruppen mit einer Dimesitylboranylgruppe substituiert ist. Das charakterisierende Merkmal dieser Verbindung ist die Anwesenheit der Diarylboranylgruppe. Verbindungen ohne diesen Substituenten sind nicht offenbart.

Aus der WO 2011/149240 ist ein Triphenylenderivat bekannt, welches mit einer Carbazolgruppe und einer Diphenyltriazingruppe substituiert ist. Das charakterisierende Merkmal dieser Verbindung ist die Anwesenheit der Triphenylengruppe. Verbindungen, die kein Triphenylen aufweisen, sind nicht offenbart.

Aus der WO 2011/046182 sind Carbazol-Arylen-Triazin-Derivate bekannt, welche am Triazin mit einer Fluorenylgruppe substituiert sind. Das charakterisierende Merkmal dieser Verbindungen ist die Anwesenheit der Fluorenylgruppe. Verbindungen, die keine Fluorenylgruppe als Substituent aufweisen, sind nicht offenbart.

WO 2012/074210 offenbart Verbindungen, in denen eine Carbazolgruppe und eine Triazingruppe über eine Fluorengruppe miteinander verknüpft sind, wobei die Verknüpfung an der 2-Position und an der 9-Position erfolgt. Es sind keine Verbindungen offenbart, in denen eine Carbazolgruppe und eine Triazingruppe über Phenylengruppen miteinander verknüpft sind.

WO 2010/131930 offenbart Verbindungen, in denen eine Carbazolgruppe und eine Triazingruppe über eine 9,9-Diphenylfluorengruppe oder eine 9-Phenylfluorengruppe miteinander verknüpft sind. Dabei erfolgt die Verknüpfung entweder über die beiden Phenylgruppen des Diphenylfluorens oder über die Phenylgruppe und die 9-Position der Phenylfluorens. Es sind keine Verbindungen offenbart, in denen eine Carbazolgruppe und eine Triazingruppe über Phenylengruppen miteinander verknüpft sind.

Generell sind bei allen diesen Verbindungen noch weitere Verbesserungen wünschenswert.

Es wurde überraschend gefunden, dass Elektrolumineszenzvorrichtungen, die Verbindungen gemäß der folgenden Formel (1) enthalten, Verbesserungen gegenüber dem Stand der Technik aufweisen, insbesondere beim Einsatz als Matrixmaterial für phosphoreszierende Dotanden.

Gegenstand der vorliegenden Erfindung ist daher eine Verbindung der folgenden Formel (1), 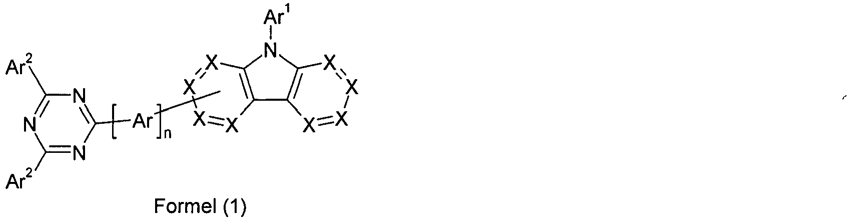 wobei für die verwendeten Symbole und Indizes gilt:
- X: ist bei jedem Auftreten gleich oder verschieden CR¹ oder N; bzw. X ist C, wenn an dieser Position die Gruppe Ar gebunden ist;
- Ar: ist bei jedem Auftreten gleich oder verschieden eine Phenylengruppe, welche durch einen oder mehrere Reste R substituiert sein kann;
- Ar¹: ist ein aromatisches Ringsystem mit 6 bis 24 aromatischen Ringatomen, welches keine kondensierten Arylgruppen mit mehr als 10 aromatischen Ringatomen aufweist und welches durch einen oder mehrere Reste R¹ substituiert sein kann, oder ist eine Dibenzofuran- oder Dibenzothiophengruppe, welche jeweils durch einen oder mehrere Reste R¹ substituiert sein kann;
- Ar²: ist bei jedem Auftreten gleich oder verschieden eine Arylgruppe, eine Biarylgruppe, eine Triarylgruppe oder Quaterarylgruppe, wobei jede einzelne der Arylgruppen in den vorstehend genannten Gruppen 6 bis 10 aromatische Ringatome hat und mit einem oder mehreren Resten R¹ substituiert sein kann, oder ist eine Dibenzofuran- oder Dibenzothiophengruppe, welche jeweils durch einen oder mehrere Reste R¹ substituiert sein kann;
- R: ist bei jedem Auftreten gleich oder verschieden H, D, F, CN, eine Arylgruppe, eine Biarylgruppe, eine Triarylgruppe oder Quateraryl-gruppe, wobei jede einzelne der Arylgruppen in den vorstehend genannten Gruppen 6 bis 10 aromatische Ringatome hat und mit einem oder mehreren Resten R¹ substituiert sein kann, oder eine über ein Kohlenstoffatom verknüpfte Carbazolgruppe, welche auch mit einem oder mehreren Reste R¹ substituiert sein kann;
- R¹: ist bei jedem Auftreten gleich oder verschieden H, D, F, CN, eine geradkettige Alkyl- oder Alkoxygruppe mit 1 bis 20 C-Atomen oder eine verzweigte oder cyclische Alkyl- oder Alkoxygruppe mit 3 bis 20 C-Atomen, wobei eine oder mehrere nicht benachbarte CH₂-Gruppen durch R²C=CR², C=C oder O ersetzt sein können und wobei ein oder mehrere H-Atome durch D oder F ersetzt sein können, oder eine Arylgruppe mit 6 bis 10 C-Atomen, die durch einen oder mehrere Reste R² substituiert sein kann, oder ist eine Dibenzofuran- oder Dibenzothiophengruppe, welche jeweils durch einen oder mehrere Reste R² substituiert sein kann, oder eine über ein Kohlenstoffatom verknüpfte Carbazolgruppe, welche auch mit einem oder mehreren Reste R² substituiert sein kann, oder eine Aralkylgruppe mit 6 bis 10 aromatischen Ringatomen, die durch einen oder mehrere Reste R² substituiert sein kann; dabei können zwei benachbarte Substituenten R¹ zusammen mit den Atomen, an die sie gebunden sind, auch miteinander ein mono- oder poly-cyclisches, aliphatisches Ringsystem bilden;
- R²: ist bei jedem Auftreten gleich oder verschieden H, D oder ein aliphatischer Kohlenwasserstoffrest mit 1 bis 20 C-Atomen oder eine Aryl-gruppe mit 6 bis 10 Ringatomen;
- n: ist 1, 2, 3 oder 4, bevorzugt 1, 2 oder 3.

Benachbarte Substituenten im Sinne der vorliegenden Erfindung sind Substituenten, die an Kohlenstoffatome gebunden sind, die direkt miteinander verknüpft sind. Dagegen sind Substituenten, die an unterschiedlichen Arylgruppen gebunden sind, keine benachbarten Substituenten im Sinne der vorliegenden Erfindung.

Bei der Arylgruppe mit 6 bis 10 aromatischen Ringatomen, wie sie für Ar², R und R¹ definiert ist, handelt es sich um Benzol oder Naphthalin, bevorzugt um Benzol. Weiterhin bevorzugt enthält das aromatische Ringsystem, wie es für Ar¹ definiert ist, überhaupt keine kondensierten Arylgruppen.

Unter einer Biarylgruppe im Sinne der vorliegenden Erfindung werden zwei direkt miteinander verknüpfte Arylgruppen verstanden, beispielsweise Biphenyl. Unter einer Triarylgruppe im Sinne der vorliegenden Erfindung werden drei direkt miteinander verknüpfte Arylgruppen verstanden, beispielsweise Terphenyl. Unter einer Quaterarylgruppe im Sinne der vorliegenden Erfindung werden vier direkt miteinander verknüpfte Arylgruppen verstanden, beispielsweise Quaterphenyl. Dabei können diese vier Arylgruppen linear oder verzweigt miteinander verknüpft sein.

Eine kondensierte Arylgruppe im Sinne der vorliegenden Erfindung ist eine eine Gruppe, in der zwei oder mehr aromatische Gruppen über eine gemeinsame Kante aneinander ankondensiert, d. h. anelliert, sind, wie beispielsweise im Naphthalin. Dagegen ist beispielsweise Fluoren keine kondensierte Arylgruppe im Sinne der vorliegenden Erfindung, da im Fluoren die beiden aromatischen Gruppen keine gemeinsame Kante aufweisen.

Ein aromatisches Ringsystem im Sinne dieser Erfindung enthält 6 bis 24 C-Atome im Ringsystem. Unter einem aromatischen Ringsystem im Sinne dieser Erfindung soll ein System verstanden werden, das nicht notwendigerweise nur Aryl- oder Heteroarylgruppen enthält, sondern in dem auch mehrere Aryl- oder Heteroarylgruppen durch eine nicht-aromatische Einheit (bevorzugt weniger als 10 % der von H verschiedenen Atome), wie z. B. ein C-Atom, verbunden sein können. So sollen beispielsweise auch Systeme wie Fluoren, 9,9'-Spirobifluoren, 9,9-Diarylfluoren, etc. als aromatische Ringsysteme im Sinne dieser Erfindung verstanden werden, und ebenso Systeme, in denen zwei oder mehrere Arylgruppen beispielsweise durch eine kurze Alkylgruppe verbunden sind. Weiterhin werden miteinander durch Einfachbindung verknüpfte Aromaten, wie zum Beispiel Biphenyl, Terphenyl oder Quaterphenyl als aromatisches Ringsystem im Sinne dieser Anmeldung bezeichnet.

Im Rahmen der vorliegenden Erfindung werden unter einem aliphatischen Kohlenwasserstoffrest bzw. einer Alkylgruppe bzw. einer Alkenylgruppe, die 1 bis 20 C-Atome enthalten kann, und in der auch einzelne H-Atome oder CH₂-Gruppen durch die oben genannten Gruppen substituiert sein können, bevorzugt die Reste Methyl, Ethyl, n-Propyl, i-Propyl, n-Butyl, i-Butyl, s-Butyl, t-Butyl, 2-Methylbutyl, n-Pentyl, s-Pentyl, Cyclopentyl, n-Hexyl, Cyclohexyl, n-Heptyl, Cycloheptyl, n-Octyl, Cyclooctyl, 2-Ethylhexyl, Trifluormethyl, Pentafluorethyl, 2,2,2-Trifluorethyl, Ethenyl, Propenyl, Butenyl, Pentenyl, Cyclopentenyl, Hexenyl, Cyclohexenyl, Heptenyl, Cycloheptenyl, Octenyl, Cyclooctenyl, Ethinyl, Propinyl, Butinyl, Pentinyl, Hexinyl, Heptinyl oder Octinyl verstanden. Unter einer Alkoxygruppe mit 1 bis 40 C-Atomen werden bevorzugt Methoxy, Trifluormethoxy, Ethoxy, n-Propoxy, i-Propoxy, n-Butoxy, i-Butoxy, s-Butoxy, t-Butoxy, n-Pentoxy, s-Pentoxy, 2-Methylbutoxy, n-Hexoxy, Cyclohexyloxy, n-Heptoxy, Cycloheptyloxy, n-Octyloxy, Cyclooctyloxy, 2-Ethylhexyloxy, Pentafluorethoxy und 2,2,2-Trifluorethoxy verstanden. Allgemein können Alkyl-, Alkenyl-, Alkinyl-, Alkoxygruppen gemäß der vorliegenden Erfindung geradkettig, verzweigt oder cyclisch sein, wobei eine oder mehrere nicht-benachbarte CH₂-Gruppen durch die oben genannten Gruppen ersetzt sein können; weiterhin können auch ein oder mehrere H-Atome durch D oder F ersetzt sein.

In einer bevorzugten Ausführungsform der Erfindung steht maximal eine Gruppe X pro Cyclus für N und die verbleibenden Gruppen X stehen gleich oder verschieden bei jedem Auftreten für CR¹. Besonders bevorzugt steht X gleich oder verschieden bei jedem Auftreten für CR¹. Bevorzugte Verbindungen der Formel (1) sind daher die Verbindungen der folgenden Formel (2), 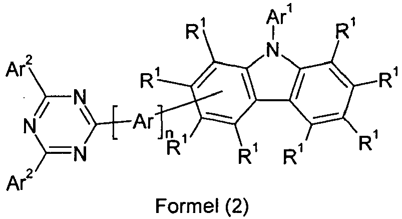 wobei die verwendeten Symbole und Indizes die oben genannten Bedeutungen aufweisen und der Rest R¹ nicht vorhanden ist, wenn an dieser Position die Gruppe Ar gebunden ist.

Die Gruppe Ar kann in der 1-, 2-, 3- oder 4-Position des Carbazols gebunden sein. In einer bevorzugten Ausführungsform der vorliegenden Erfindung ist die Gruppe Ar in der 1-, 2- bzw. 3-Position des Carbazols gebunden. Bevorzugt sind daher die Verbindungen der folgenden Formeln (2a), (2b) und (2c), 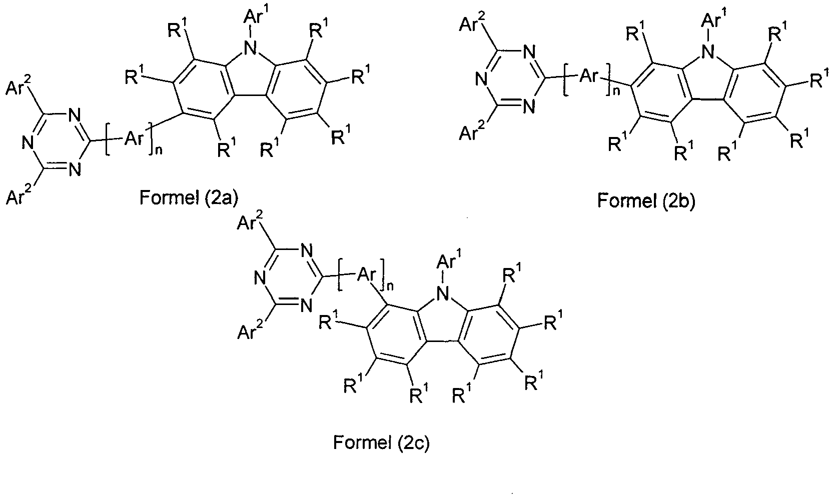 wobei die verwendeten Symbole und Indizes die oben genannten Bedeutungen aufweisen.

Besonders bevorzugt sind die Verbindungen der oben genannten Formeln (2a) und (2b).

In einer bevorzugten Ausführungsform der Erfindung ist n = 1, 2 oder 3 und Ar steht für eine Phenylengruppe, welche durch einen oder mehrere Reste R substituiert sein kann. Dabei steht Ar gleich oder verschieden bei jedem Auftreten für eine Gruppe ausgewählt aus den folgenden Formeln (3), (4) oder (5), 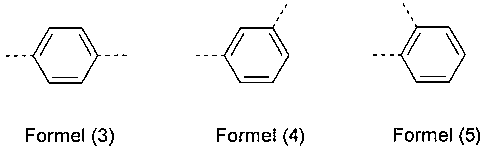 wobei die gestrichelte Bindung jeweils die Verknüpfung dieser Gruppen andeutet und jede dieser Gruppen durch einen oder mehrere Reste R substituiert sein kann.

Insbesondere bevorzugt steht die Gruppe -(Ar)ₙ- für eine Gruppe ausgewählt aus den Formeln (6) bis (18), 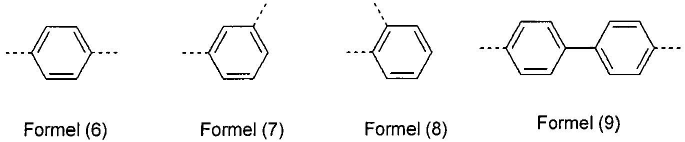 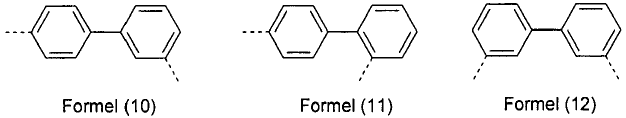 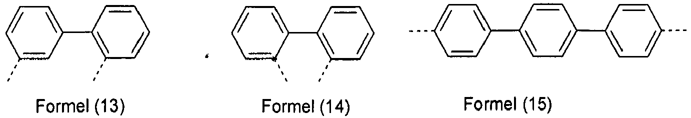 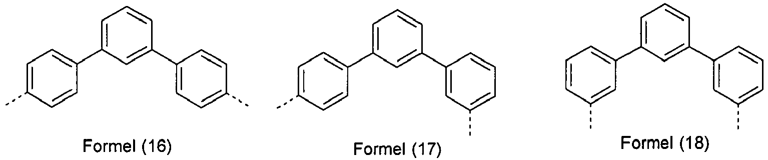 wobei die gestrichelte Bindung jeweils die Verknüpfung dieser Gruppen andeutet, jede dieser Gruppen auch durch einen oder mehrere Reste R substituiert sein kann und R die oben genannten Bedeutungen aufweist.

Der Rest R, welcher an Ar bzw. an den oben ausgeführten bevorzugten Ausführungsformen von Ar gebunden ist, ist dabei bevorzugt gleich oder verschieden bei jedem Auftreten ausgewählt aus der Gruppe bestehend aus H, D, F, CN, Phenyl, Biphenyl oder Terphenyl, wobei jede der Arylgruppen in den vorstehend genannten Gruppen mit einem oder mehreren Resten R¹ substituiert sein kann und R¹ bevorzugt gleich oder verschieden bei jedem Auftreten für H oder eine Alkylgruppe mit 1 bis 5 C-Atomen steht, oder eine über ein Kohlenstoffatom verknüpfte Carbazolgruppe, welche auch mit einem oder mehreren Reste R¹ substituiert sein kann. Besonders bevorzugt ist R ausgewählt aus der Gruppe bestehend aus H, Phenyl, Biphenyl oder Terphenyl oder eine über ein Kohlenstoffatom verknüpfte N-Phenylcarbazolgruppe.

In einer weiterhin bevorzugten Ausführungsform der Erfindung ist Ar¹ ausgewählt aus der Gruppe bestehend aus Phenyl, Biphenyl, Terphenyl oder Quaterphenyl, welches jeweils mit einem oder mehreren Resten R¹ substituiert sein kann.

In nochmals einer weiteren bevorzugten Ausführungsform der Erfindung ist Ar² gleich oder verschieden bei jedem Auftreten ausgewählt aus der Gruppe bestehend aus Phenyl, Biphenyl, Terphenyl oder Quaterphenyl, welches jeweils mit einem oder mehreren Resten R¹ substituiert sein kann.

In nochmals einer weiteren bevorzugten Ausführungsform der Erfindung ist R¹ bei jedem Auftreten gleich oder verschieden ausgewählt aus der Gruppe bestehend aus H, F, einer geradkettigen Alkylgruppe mit 1 bis 10 C-Atomen oder einer verzweigten oder cyclischen Alkylgruppe mit 3 bis 10 C-Atomen, wobei eine oder mehrere nicht benachbarte CH₂-Gruppen durch R²C=CR² ersetzt sein können und wobei ein oder mehrere H-Atome durch F ersetzt sein können, oder eine Arylgruppe mit 6 bis 10 C-Atomen, die durch einen oder mehrere Reste R² substituiert sein kann, oder ist eine Dibenzofuran- oder Dibenzothiophengruppe, welche jeweils durch einen oder mehrere Reste R² substituiert sein kann, oder eine über ein Kohlenstoffatom verknüpfte Carbazolgruppe, welche auch mit einem oder mehreren Reste R² substituiert sein kann.

Besonders bevorzugt ist R¹ bei jedem Auftreten gleich oder verschieden ausgewählt aus der Gruppe bestehend aus H, einer geradkettigen Alkylgruppe mit 1 bis 5 C-Atomen oder einer verzweigten oder cyclischen Alkylgruppe mit 3 bis 5 C-Atomen, oder eine Arylgruppe mit 6 bis 10 C-Atomen, die durch einen oder mehrere Reste R² substituiert sein kann.

Die oben genannten Ausführungsformen der Erfindung sind beliebig miteinander kombinierbar. Besonders bevorzugt werden die oben als bevorzugt genannten Ausführungsformen miteinander kombiniert.

In einer bevorzugten Ausführungsform der Erfindung gilt also für die Verbindungen der Formel (1), (2), (2a), (2b) und (2c) für die verwendeten Symbole und Indizes Folgendes:
- Ar: steht gleich oder verschieden bei jedem Auftreten für eine Gruppe der oben aufgeführten Formel (3), (4) oder (5), wobei n = 1, 2 oder 3 ist;
- R: ist gleich oder Verschieden bei jedem Auftreten ausgewählt aus der Gruppe bestehend aus H, D, F, CN, Phenyl, Biphenyl oder Terphenyl, wobei jede der Arylgruppen in den vorstehend genannten Gruppen mit einem oder mehreren Resten R¹ substituiert sein kann und R¹ bevorzugt gleich oder verschieden bei jedem Auftreten für H oder eine Alkylgruppe mit 1 bis 5 C-Atomen steht, oder eine über ein Kohlenstoffatom verknüpfte Carbazolgruppe, welche auch mit einem oder mehreren Reste R¹ substituiert sein kann.
- Ar¹: ist ausgewählt aus der Gruppe bestehend aus Phenyl, Biphenyl, Terphenyl oder Quaterphenyl, welches jeweils mit einem oder mehreren Resten R¹ substituiert sein kann;
- Ar²: ist gleich oder verschieden bei jedem Auftreten ausgewählt aus der Gruppe bestehend aus Phenyl, Biphenyl, Terphenyl oder Quaterphenyl, welches jeweils mit einem oder mehreren Resten R¹ substituiert sein kann;
- R¹: ist bei jedem Auftreten gleich oder verschieden ausgewählt aus der Gruppe bestehend aus H, D, F, einer geradkettigen Alkylgruppe mit 1 bis 10 C-Atomen oder einer verzweigten oder cyclischen Alkylgruppe mit 3 bis 10 C-Atomen, wobei eine oder mehrere nicht benachbarte CH₂-Gruppen durch R²C=CR² ersetzt sein können und wobei ein oder mehrere H-Atome durch F ersetzt sein können, oder eine Arylgruppe mit 6 bis 10 C-Atomen, die durch einen oder mehrere Reste R² substituiert sein kann, oder ist eine Dibenzofuran- oder Dibenzothiophengruppe, welche jeweils durch einen oder mehrere Reste R² substituiert sein kann; oder eine über ein Kohlenstoffatom verknüpfte Carbazolgruppe, welche auch mit einem oder mehreren Reste R² substituiert sein kann.

In einer besonders bevorzugten Ausführungsform der Erfindung gilt also für die Verbindungen der Formel (1), (2), (2a), (2b) und (2c) für die verwendeten Symbole und Indizes Folgendes:

-(Ar)ₙ-

steht für eine Gruppe gemäß einer der oben aufgeführten Formeln (6) bis (18);
- R: ist gleich oder verschieden bei jedem Auftreten ausgewählt aus der Gruppe bestehend aus H, Phenyl, Biphenyl, Terphenyl oder eine über ein Kohlenstoffatom verknüpfte N-Phenylcarbazolgruppe;
- Ar¹: ist ausgewählt aus der Gruppe bestehend aus Phenyl, Biphenyl, Terphenyl oder Quaterphenyl, welches jeweils mit einem oder mehreren Resten R¹ substituiert sein kann;
- Ar²: ist gleich oder verschieden bei jedem Auftreten ausgewählt aus der Gruppe bestehend aus Phenyl, Biphenyl, Terphenyl oder Quaterphenyl, welches jeweils mit einem oder mehreren Resten R¹ substituiert sein kann;
- R¹: ist bei jedem Auftreten gleich oder verschieden ausgewählt aus der Gruppe bestehend aus H, einer geradkettigen Alkylgruppe mit 1 bis 5 C-Atomen oder einer verzweigten oder cyclischen Alkylgruppe mit 3 bis 5 C-Atomen, oder eine Arylgruppe mit 6 bis 10 C-Atomen, die durch einen oder mehrere Reste R² substituiert sein kann.

Beispiele für erfindungsgemäße Verbindungen sind die nachstehend gezeigten Strukturen.

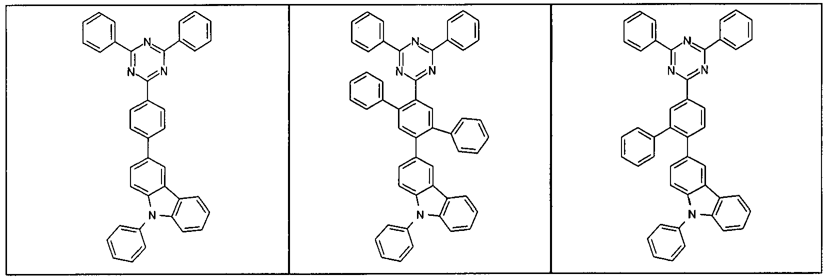

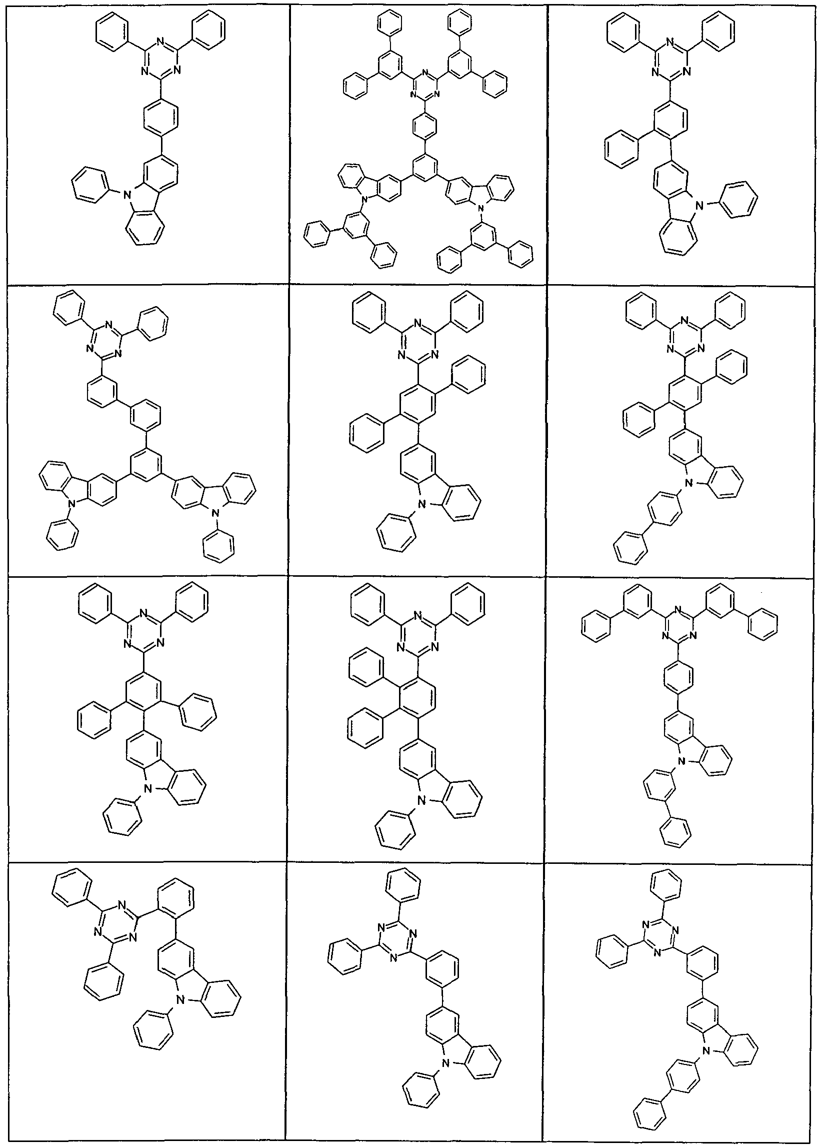

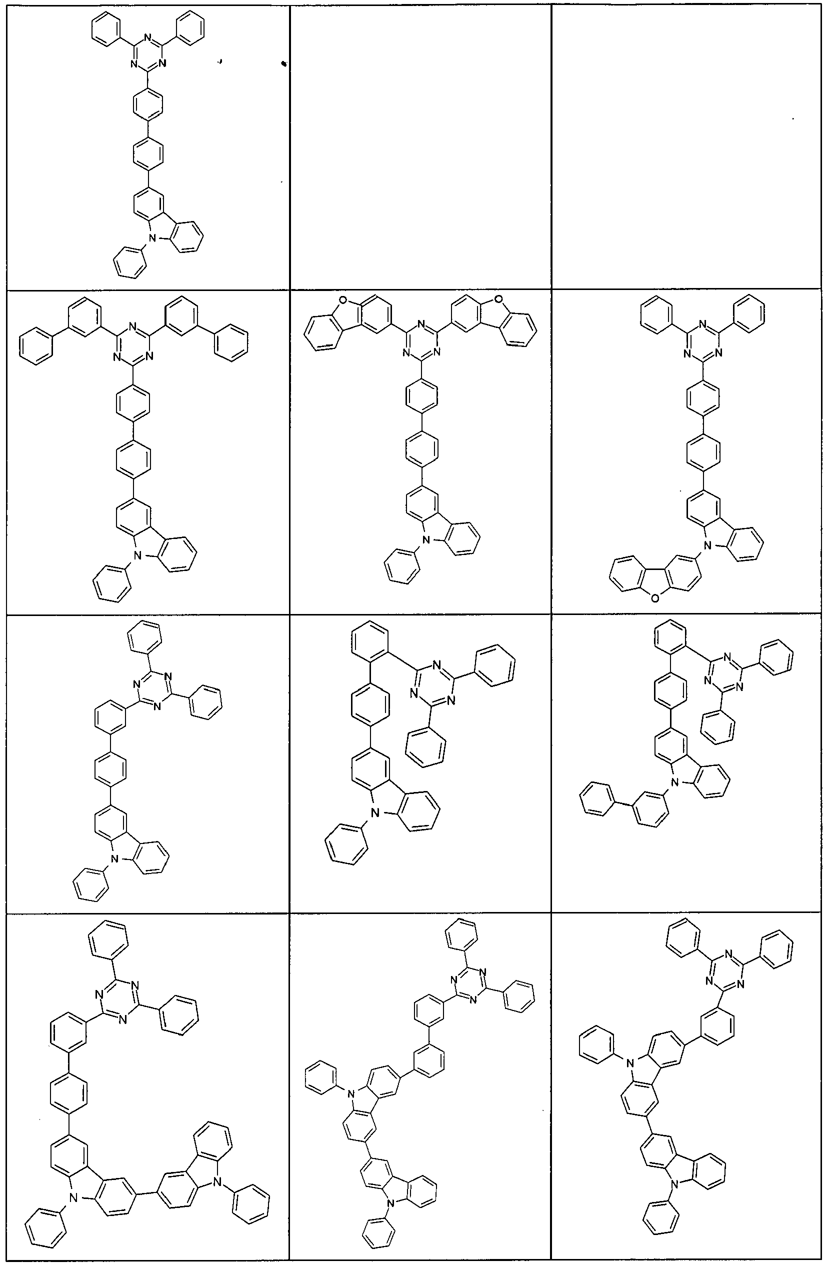

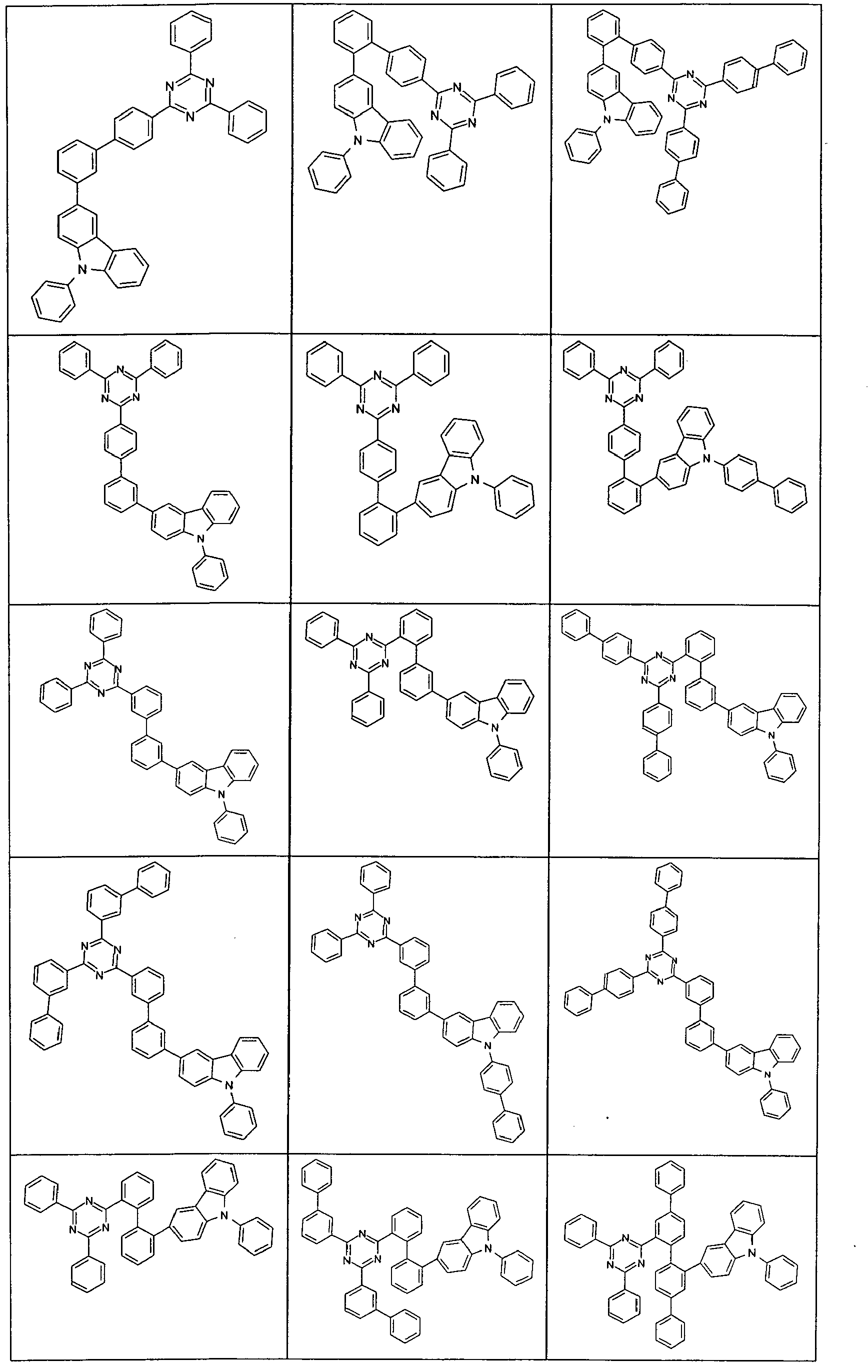

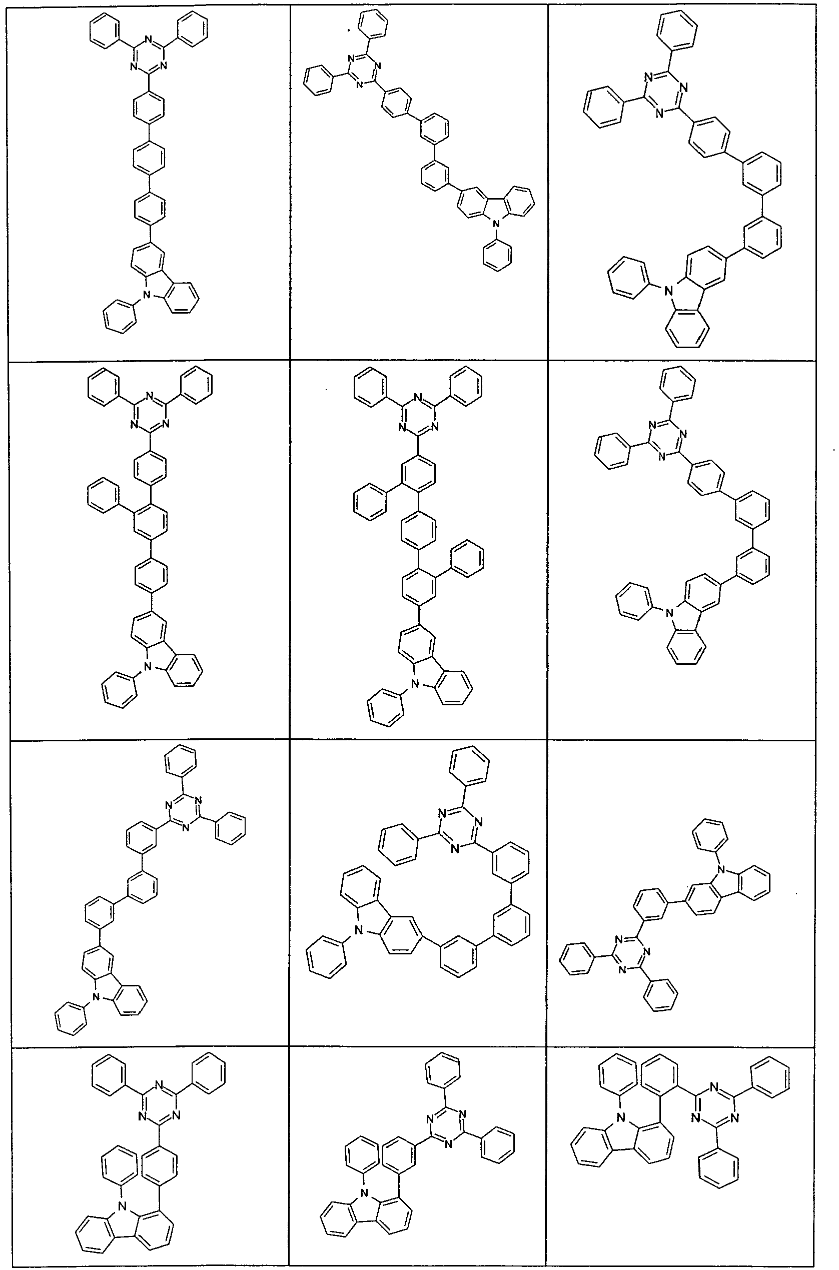

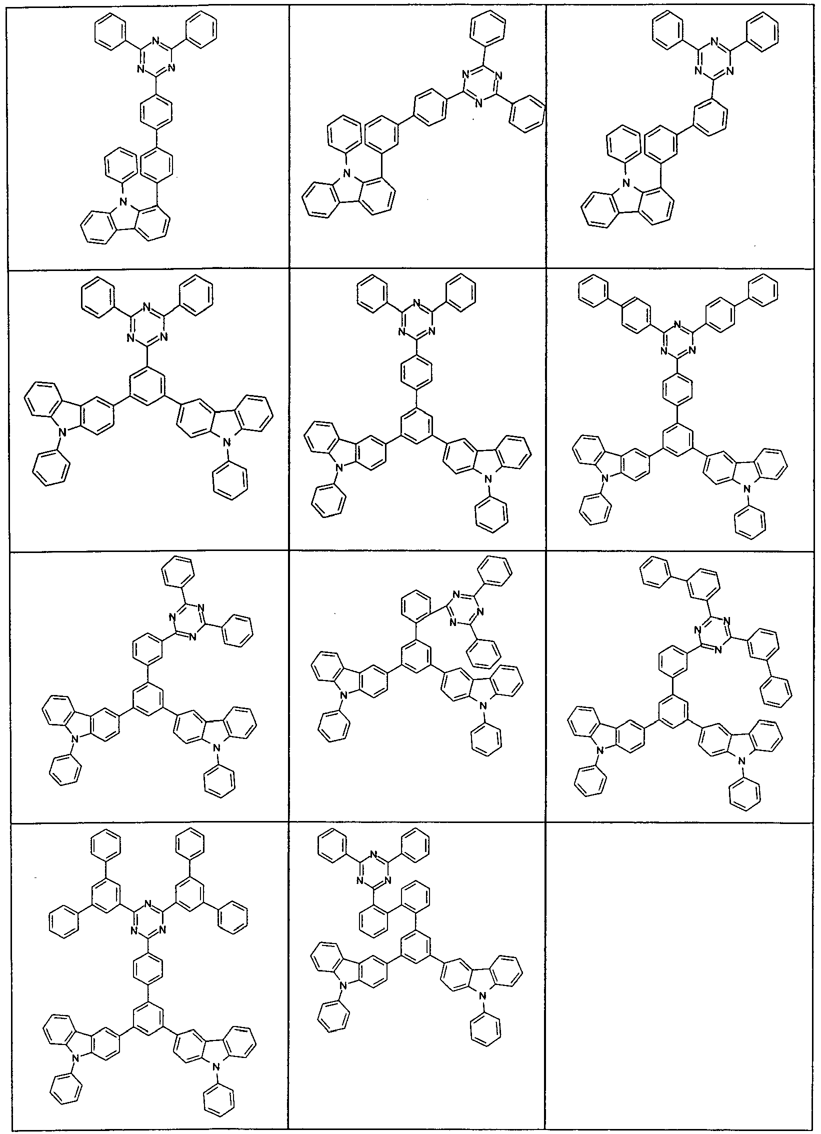

Die erfindungsgemäßen Verbindungen können nach dem Fachmann bekannten Syntheseschritten, wie z. B. Bromierung, Suzuki-Kupplung, Ullmann-Kupplung, Hartwig-Buchwald-Kupplung, etc., dargestellt werden. Ein geeignetes Syntheseverfahren ist allgemein im folgenden Schema 1 dargestellt:

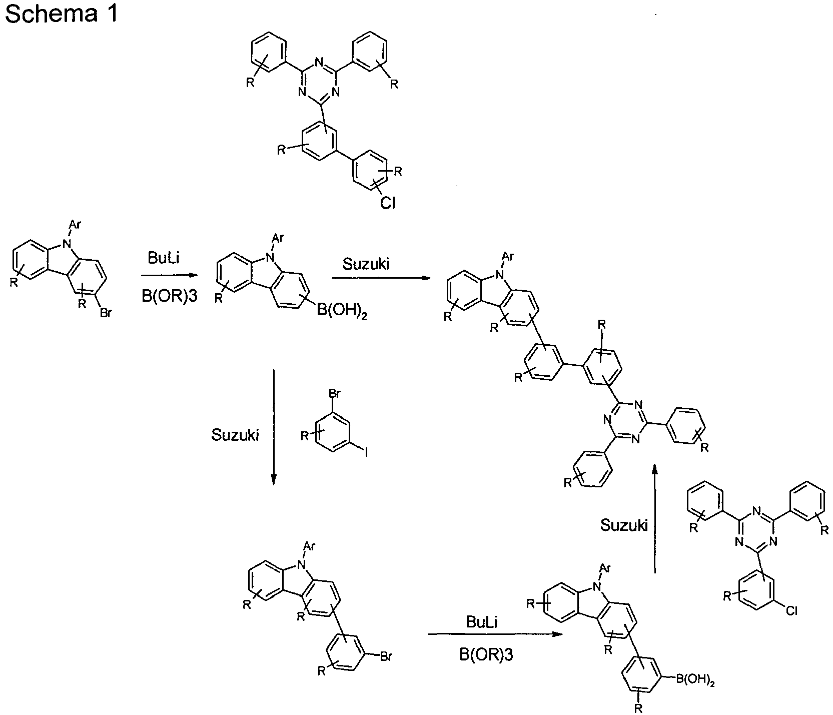

Ein weiterer Gegenstand der Erfindung ist daher ein Verfahren zur Herstellung einer erfindungsgemäßen Verbindung, dadurch gekennzeichnet, dass die Einheit (Ar²)₂Triazin-(Ar)ₙ durch eine Suzuki-Kupplung eingeführt wird.

Ein weiterer Gegenstand der vorliegenden Erfindung sind Mischungen enthaltend mindestens eine erfindungsgemäße Verbindung und mindestens eine weitere Verbindung. Die weitere Verbindung kann beispielsweise ein fluoreszierender oder phosphoreszierender Dotand sein, wenn die erfindungsgemäße Verbindung als Matrixmaterial verwendet wird, insbesondere ein phosphoreszierender Dotand. Geeignete Dotanden sind unten im Zusammenhang mit den organischen Elektrolumineszenzvorrichtungen aufgeführt und sind auch für die erfindungsgemäßen Mischungen bevorzugt.

Für die Verarbeitung aus Lösung bzw. aus flüssiger Phase, beispielsweise durch Spin-Coating oder durch Druckverfahren, sind Lösungen bzw. Formulierungen der erfindungsgemäßen Verbindungen bzw. Mischungen erforderlich. Es kann bevorzugt sein, Mischungen aus zwei oder mehr Lösemitteln zu verwenden. Geeignete und bevorzugte Lösemittel sind beispielsweise Toluol, Anisol, o-, m- oder p-Xylol, Methylbenzoat, Dimethylanisol, Mesitylen, Tetralin, Veratrol, THF, Methyl-THF, THP, Chlorbenzol, Dioxan oder Mischungen dieser Lösemittel.

Ein weiterer Gegenstand der vorliegenden Erfindung ist daher eine Formulierung, insbesondere eine Lösung, eine Suspension oder eine Miniemulsion, enthaltend mindestens eine erfindungsgemäße Verbindung oder Mischung und ein oder mehrere Lösemittel, insbesondere organische Lösemittel. Wie solche Lösungen hergestellt werden können, ist dem Fachmann bekannt und beispielsweise in WO 2002/072714, WO 2003/019694 und der darin zitierten Literatur beschrieben.

Die erfindungsgemäßen Verbindungen und Mischungen eignen sich für die Verwendung in einer elektronischen Vorrichtung. Dabei wird unter einer elektronischen Vorrichtung eine Vorrichtung verstanden, welche mindestens eine Schicht enthält, die mindestens eine organische Verbindung enthält. Das Bauteil kann dabei aber auch anorganische Materialien enthalten oder auch Schichten, welche vollständig aus anorganischen Materialien aufgebaut sind.

Ein weiterer Gegenstand der vorliegenden Erfindung ist daher die Verwendung der erfindungsgemäßen Verbindungen oder Mischungen in einer elektronischen Vorrichtung, insbesondere in einer organischen Elektrolumineszenzvorrichtung.

Nochmals ein weiterer Gegenstand der vorliegenden Erfindung ist eine elektronische Vorrichtung enthaltend mindestens eine der oben ausgeführten erfindungsgemäßen Verbindungen oder Mischungen. Dabei gelten die oben für die Verbindung ausgeführten Bevorzugungen auch für die elektronischen Vorrichtungen.

Die elektronische Vorrichtung ist bevorzugt ausgewählt aus der Gruppe bestehend aus organischen Elektrolumineszenzvorrichtungen (OLEDs, PLEDs), organischen integrierten Schaltungen (O-ICs), organischen Feld-Effekt-Transistoren (O-FETs), organischen Dünnfilmtransistoren (O-TFTs), organischen lichtemittierenden Transistoren (O-LETs), organischen Solarzellen (O-SCs), organischen farbstoff-sensibilisierten Solarzellen, organischen optischen Detektoren, organischen Photorezeptoren, organischen Feld-Quench-Devices (O-FQDs), lichtemittierenden elektrochemischen Zellen (LECs), organischen Laserdioden (O-Laser) und "organic plasmon emitting devices" (D. M. Koller et al., Nature Photonics 2008, 1-4), bevorzugt organischen Elektrolumineszenzvorrichtungen (OLEDs, PLEDs), insbesondere phosphoreszierenden OLEDs.

Die organische Elektrolumineszenzvorrichtung enthält Kathode, Anode und mindestens eine emittierende Schicht. Außer diesen Schichten kann sie noch weitere Schichten enthalten, beispielsweise jeweils eine oder mehrere Lochinjektionsschichten, Lochtransportschichten, Lochblockierschichten, Elektronentransportschichten, Elektroneninjektionsschichten, Exzitonenblockierschichten, Elektronenblockierschichten und/oder Ladungserzeugungsschichten (Charge-Generation Layers). Ebenso können zwischen zwei emittierende Schichten Zwischenschichten (Interlayer) eingebracht sein, welche beispielsweise eine exzitonenblockierende Funktion aufweisen. Es sei aber darauf hingewiesen, dass nicht notwendigerweise jede dieser Schichten vorhanden sein muss. Dabei kann die organische Elektrolumineszenzvorrichtung eine emittierende Schicht enthalten, oder sie kann mehrere emittierende Schichten enthalten. Wenn mehrere Emissionsschichten vorhanden sind, weisen diese bevorzugt insgesamt mehrere Emissionsmaxima zwischen 380 nm und 750 nm auf, so dass insgesamt weiße Emission resultiert, d. h. in den emittierenden Schichten werden verschiedene emittierende Verbindungen verwendet, die fluoreszieren oder phosphoreszieren können. Insbesondere bevorzugt sind Systeme mit drei emittierenden Schichten, wobei die drei Schichten blaue, grüne und orange oder rote Emission zeigen (für den prinzipiellen Aufbau siehe z. B. WO 2005/011013). Es kann sich dabei um fluoreszierende oder um phosphoreszierende Emissionsschichten handeln oder um Hybrid-Systeme, bei denen fluoreszierende und phosphoreszierende Emissionsschichten miteinander kombiniert werden.

Die erfindungsgemäße Verbindung gemäß den oben aufgeführten Ausführungsformen kann dabei in unterschiedlichen Schichten eingesetzt werden, je nach genauer Struktur. Bevorzugt ist eine organische Elektrolumineszenzvorrichtung, enthaltend eine Verbindung gemäß Formel (1) oder gemäß den bevorzugten Ausführungsformen als Matrixmaterial für fluoreszierende oder phosphoreszierende Emitter, insbesondere für phosphoreszierende Emitter, und/oder in einer Elektronentransportschicht und/oder in einer elektronenblockierenden bzw. exzitonenblockierenden Schicht und/oder in einer Lochtransportschicht, je nach genauer Substitution. Dabei gelten die oben aufgeführten bevorzugten Ausführungsformen auch für die Verwendung der Materialien in organischen elektronischen Vorrichtungen.

In einer bevorzugten Ausführungsform der Erfindung wird die Verbindung gemäß Formel (1) bzw. gemäß den bevorzugten Ausführungsformen als Matrixmaterial für eine fluoreszierende oder phosphoreszierende Verbindung, insbesondere für eine phosphoreszierende Verbindung, in einer emittierenden Schicht eingesetzt. Dabei kann die organische Elektrolumineszenzvorrichtung eine emittierende Schicht enthalten, oder sie kann mehrere emittierende Schichten enthalten, wobei mindestens eine emittierende Schicht mindestens eine erfindungsgemäße Verbindung als Matrixmaterial enthält.

Wenn die Verbindung gemäß Formel (1) bzw. gemäß den bevorzugten Ausführungsformen als Matrixmaterial für eine emittierende Verbindung in einer emittierenden Schicht eingesetzt wird, wird sie bevorzugt in Kombination mit einem oder mehreren phosphoreszierenden Materialien (Triplettemitter) eingesetzt. Unter Phosphoreszenz im Sinne dieser Erfindung wird die Lumineszenz aus einem angeregten Zustand mit Spinmultiplizität > 1, insbesondere aus einem angeregten Triplettzustand. Im Sinne dieser Anmeldung sollen alle lumineszierenden Übergangsmetallkomplexe und lumineszierenden Lanthanidkomplexe, insbesondere alle Iridium-, Platin- und Kupferkomplexe als phosphoreszierende Verbindungen angesehen werden.

Die Mischung aus der Verbindung gemäß Formel (1) bzw. gemäß den bevorzugten Ausführungsformen und der emittierenden Verbindung enthält zwischen 99 und 1 Vol.-%, vorzugsweise zwischen 98 und 10 Vol.-%, besonders bevorzugt zwischen 97 und 60 Vol.-%, insbesondere zwischen 95 und 80 Vol.-% der Verbindung gemäß Formel (1) bzw. gemäß den bevorzugten Ausführungsformen bezogen auf die Gesamtmischung aus Emitter und Matrixmaterial. Entsprechend enthält die Mischung zwischen 1 und 99 Vol.-%, vorzugsweise zwischen 2 und 90 Vol.-%, besonders bevorzugt zwischen 3 und 40 Vol.-%, insbesondere zwischen 5 und 20 Vol.-% des Emitters bezogen auf die Gesamtmischung aus Emitter und Matrixmaterial.

Eine weitere bevorzugte Ausführungsform der vorliegenden Erfindung ist der Einsatz der Verbindung gemäß Formel (1) bzw. gemäß den bevorzugten Ausführungsformen als Matrixmaterial für einen phosphoreszierenden Emitter in Kombination mit einem weiteren Matrixmaterial. Besonders geeignete Matrixmaterialien, welche in Kombination mit den Verbindungen gemäß Formel (1) bzw. gemäß den bevorzugten Ausführungsformen eingesetzt werden können, sind aromatische Ketone, aromatische Phosphinoxide oder aromatische Sulfoxide oder Sulfone, z. B. gemäß WO 2004/013080, WO 2004/093207, WO 2006/005627 oder WO 2010/006680, Triarylamine, Carbazolderivate, z. B. CBP (N,N-Bis-carbazolylbiphenyl) oder die in WO 2005/039246, US 2005/0069729, JP 2004/288381, EP 1205527 oder WO 2008/086851 offenbarten Carbazolderivate, Indolocarbazolderivate, z. B. gemäß WO 2007/063754 oder WO 2008/056746, Indenocarbazolderivate, z. B. gemäß WO 2010/136109 und WO 2011/000455, Azacarbazolderivate, z. B. gemäß EP 1617710, EP 1617711, EP 1731584, JP 2005/347160, bipolare Matrixmaterialien, z. B. gemäß WO 2007/137725, Silane, z. B. gemäß WO 005/111172, Azaborole oder Boronester, z. B. gemäß WO 2006/117052, Triazinderivate, z. B. gemäß WO 2010/015306, WO 2007/063754 oder WO 2008/056746, Zinkkomplexe, z. B. gemäß EP 652273 oder WO 2009/062578, Diazasilol- bzw. Tetraazasilol-Derivate, z. B. gemäß WO 2010/054729, Diazaphosphol-Derivate, z. B. gemäß WO 2010/054730, überbrückte Carbazol-Derivate, z. B. gemäß US 2009/0136779, WO 2010/050778, WO 2011/042107, WO 2011/088877 oder gemäß der nicht offen gelegten Anmeldung EP 11003232.3, Triphenylenderivaten, z. B. gemäß WO 2012/048781, oder Lactame, z. B. gemäß WO 2011/116865 oder WO 2011/137951. Ebenso kann ein weiterer phosphoreszierender Emitter, welcher kürzerwellig als der eigentliche Emitter emittiert, als Co-Host in der Mischung vorhanden sein.

Als phosphoreszierende Verbindungen (= Triplettemitter) eignen sich insbesondere Verbindungen, die bei geeigneter Anregung Licht, vorzugsweise im sichtbaren Bereich, emittieren und außerdem mindestens ein Atom der Ordnungszahl größer 20, bevorzugt größer 38 und kleiner 84, besonders bevorzugt größer 56 und kleiner 80 enthalten, insbesondere ein Metall mit dieser Ordnungszahl. Bevorzugt werden als Phosphoreszenzemitter Verbindungen, die Kupfer, Molybdän, Wolfram, Rhenium, Ruthenium, Osmium, Rhodium, Iridium, Palladium, Platin, Silber, Gold oder Europium enthalten, verwendet, insbesondere Verbindungen, die Iridium oder Platin enthalten. Im Sinne der vorliegenden Erfindung werden alle lumineszierenden Verbindungen, die die oben genannten Metalle enthalten, als phosphoreszierende Verbindungen angesehen.

Beispiele der oben beschriebenen Emitter können den Anmeldungen WO 00/70655, WO 2001/41512, WO 2002/02714, WO 2002/15645, EP 1191613, EP 1191612, EP 1191614, WO 05/033244, WO 05/019373, US 2005/0258742, WO 2009/146770, WO 2010/015307, WO 2010/031485, WO 2010/054731, WO 2010/054728, WO 2010/086089, WO 2010/099852, WO 2010/102709, WO 2011/032626, WO 2011/066898, WO 2011/157339 oder WO 2012/007086 entnommen werden. Generell eignen sich alle phosphoreszierenden Komplexe, wie sie gemäß dem Stand der Technik für phosphoreszierende OLEDs verwendet werden und wie sie dem Fachmann auf dem Gebiet der organischen Elektrolumineszenz bekannt sind, und der Fachmann kann ohne erfinderisches Zutun weitere phosphoreszierende Komplexe verwenden.

In einer weiteren Ausführungsform der Erfindung enthält die erfindungsgemäße organische Elektrolumineszenzvorrichtung keine separate Lochinjektionsschicht und/oder Lochtransportschicht und/oder Lochblockierschicht und/oder Elektronentransportschicht, d. h. die emittierende Schicht grenzt direkt an die Lochinjektionschicht oder die Anode an, und/ oder die emittierende Schicht grenzt direkt an die Elektronentransportschicht oder die Elektroneninjektionsschicht oder die Kathode an, wie zum Beispiel in WO 2005/053051 beschrieben. Weiterhin ist es möglich, einen Metallkomplex, der gleich oder ähnlich dem Metallkomplex in der emittierenden Schicht ist, direkt angrenzend an die emittierende Schicht als Lochtransport- bzw. Lochinjektionsmaterial zu verwenden, wie z. B. in WO 2009/030981 beschrieben.

Weiterhin ist es möglich, die erfindungsgemäßen Verbindungen in einer Lochtransportschicht oder in einer Lochinjektionsschicht oder in einer Exzitonen- bzw. Elektronenblockierschicht einzusetzen.

In den weiteren Schichten der erfindungsgemäßen organischen Elektrolumineszenzvorrichtung können alle Materialien verwendet werden, wie sie üblicherweise gemäß dem Stand der Technik eingesetzt werden. Der Fachmann kann daher ohne erfinderisches Zutun alle für organische Elektrolumineszenzvorrichtungen bekannten Materialien in Kombination mit den erfindungsgemäßen Verbindungen gemäß Formel (1) bzw. gemäß den bevorzugten Ausführungsformen.

Weiterhin bevorzugt ist eine organische Elektrolumineszenzvorrichtung, dadurch gekennzeichnet, dass eine oder mehrere Schichten mit einem Sublimationsverfahren beschichtet werden. Dabei werden die Materialien in Vakuum-Sublimationsanlagen bei einem Anfangsdruck kleiner 10⁻⁵ mbar, bevorzugt kleiner 10⁻⁶ mbar aufgedampft. Es ist aber auch möglich, dass der Anfangsdruck noch geringer oder höher ist, beispielsweise kleiner 10⁻⁷ mbar.

Bevorzugt ist ebenfalls eine organische Elektrolumineszenzvorrichtung, dadurch gekennzeichnet, dass eine oder mehrere Schichten mit dem OVPD (Organic Vapour Phase Deposition) Verfahren oder mit Hilfe einer Trägergassublimation beschichtet werden. Dabei werden die Materialien bei einem Druck zwischen 10⁻⁵ mbar und 1 bar aufgebracht. Ein Spezialfall dieses Verfahrens ist das OVJP (Organic Vapour Jet Printing) Verfahren, bei dem die Materialien direkt durch eine Düse aufgebracht und so strukturiert werden (z. B. M. S. Arnold et al., Appl. Phys. Lett. 2008, 92, 053301).

Weiterhin bevorzugt ist eine organische Elektrolumineszenzvorrichtung, dadurch gekennzeichnet, dass eine oder mehrere Schichten aus Lösung, wie z. B. durch Spincoating, oder mit einem beliebigen Druckverfahren, wie z. B. Ink-Jet Druck (Tintenstrahldruck), LITI (Light Induced Thermal Imaging, Thermotransferdruck), Siebdruck, Flexodruck, Offsetdruck oder Nozzle-Printing hergestellt werden. Hierfür sind lösliche Verbindungen nötig, welche beispielsweise durch geeignete Substitution erhalten werden.

Weiterhin sind Hybridverfahren möglich, bei denen beispielsweise eine oder mehrere Schichten aus Lösung aufgebracht werden und eine oder mehrere weitere Schichten aufgedampft werden. So ist es beispielsweise möglich, die emittierende Schicht aus Lösung aufzubringen und die Elektronentransportschicht aufzudampfen.

Diese Verfahren sind dem Fachmann generell bekannt und können von ihm ohne erfinderisches Zutun auf organische Elektrolumineszenzvorrichtungen enthaltend die erfindungsgemäßen Verbindungen angewandt werden.

Die erfindungsgemäßen Verbindungen weisen bei Verwendung in organischen Elektrolumineszenzvorrichtungen einen oder mehrere der folgenden überraschenden Vorteile gegenüber dem Stand der Technik auf:
1. Die Leistungseffizienz entsprechender Vorrichtungen wird höher im Vergleich zu Systemen gemäß dem Stand der Technik.
2. Die Stabilität entsprechender Vorrichtungen wird höher im Vergleich zu Systemen gemäß dem Stand der Technik, was sich vor allem in einer höheren Lebensdauer zeigt.
3. Die erfindungsgemäßen organische Elektroluminszenzvorrichtungen weisen eine verringerte Betriebsspannung auf.
4. Die erfindungsgemäßen Verbindungen sind einfach und in hoher Ausbeute synthetisch zugänglich.
5. Die erfindungsgemäßen Verbindungen weisen eine sehr gute thermische Stabilität und somit eine hohe Sublimationsstabilität auf.

Die Erfindung wird nun durch die nachfolgenden Beispiele näher erläutert, ohne sie dadurch einschränken zu wollen.

### Beispiele

Die nachfolgenden Synthesen werden, sofern nicht anders angegeben, unter einer Schutzgasatmosphäre in getrockneten Lösungsmitteln durchgeführt. Die Lösungsmittel und Reagenzien können z. B. von Sigma-ALDRICH bzw. ABCR bezogen werden. Zu den literaturbekannten Verbindungen sind jeweils auch die entsprechenden CAS-Nummern angegeben.

### Beispiel 1: 2,4-Bis-(4-tert-butyl-phenyl)-6-chlor-[1,3,5]triazin

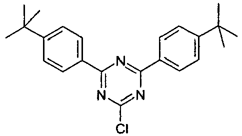

5.7 g Magnesium (234.6 mmol) werden in einem 500 mL Vierhalskolben vorgelegt, und eine Lösung aus 50 g Brom-4-tert-butyl-phenyl (234.6 mmol) in 200 ml THF wird langsam zugetropft. Die Reaktionsmischung wird 1.5 h zum Sieden erhitzt und anschließend auf Raumtemperatur abgekühlt. In einem zweiten Kolben wird Cyanchlorid (18.8 g, 102 mmol) in 200 ml THF vorgelegt und auf 0 °C gekühlt. Bei dieser Temperatur wird das abgekühlte Grignard-Reagenz zugetropft und 12 h bei Raumtemperatur gerührt. Nach dieser Zeit wird die Reaktionsmischung mit 150 ml HCl versetzt und die wässrige Phase dreimal mit Dichlormethan extrahiert. Die vereinigten organischen Phasen werden mit Wasser gewaschen, über Na₂SO₄ getrocknet und eingeengt. Der Rückstand wird aus Ethanol umkristallisiert. Die Ausbeute beträgt 31 g (81.6 mmol, 80%).

### Beispiel 2: 2,4-Bis-(3-brom-phenyl)-6-phenyl-[1,3,5]triazin

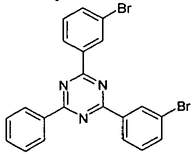

49 ml (392 mmol) Benzoylchlorid, 52.3 g (392 mmol) AlCl₃ und 8.5 ml Thionylchlorid werden unter Schutzgasatmosphäre in 500 ml 1,2-Dichlorbenzol vorgelegt. Über einen Tropftrichter werden bei Raumtemperatur 150 g (824 mmol) 3-Brombenzonitril, gelöst in 300 ml 1,2-Dichlorbenzol zu dieser Lösung zugetropft, anschließend 1 h bei 100 °C gerührt, dann 18 h bei 40 °C gerührt. Nach dieser Zeit wird das Reaktionsgemisch mit 1.5 L Methanol versetzt und der Rückstand getrennt. Der Rückstand wird mit Methanol heiß ausgerührt. Man erhält 59 g (126 mmol) (32 %) des Produkts.

### Beispiel 3:2,4-Bis-biphenyl-3-yl-6-chloro-[1,3,5]triazin

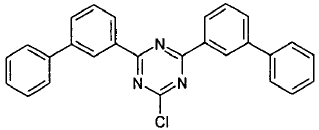

5.2 g Magnesium (0,215 mol) werden in einem 500 mL Vierhalskolben vorgelegt, und eine Lösung aus 50 g Brombiphenyl (214 mmol) in 200 ml THF wird langsam zugetropft. Die Reaktionsmischung wird 1.5 h zum Sieden erhitzt und anschließend auf Raumtemperatur abgekühlt. In einem zweiten Kolben wird Cyanchlorid (17.2 g, 93 mmol) in 150 ml THF vorgelegt und auf 0 °C gekühlt. Bei dieser Temperatur dann das abgekühlte Grignard Reagenz zugetropft und 12 h bei RT gerührt. Nach dieser Zeit wird die Reaktionsmischung mit 150 HCl versetzt und die wässrige Phase dreimal mit Dichlormethan extrahiert. Die vereinigten organischen Phasen werden mit Wasser gewaschen, über über Na₂SO₄ getrocknet und eingeengt. Der Rückstand wird aus EtOH umkristallisiert. Die Ausbeute beträgt 32.8 g (78 mmol, 84%).

### Beispiel 4: 2-Chlor-4,6-bis-[1,1';3',1"]terphenyl-5'-yl-[1,3,5]triazin

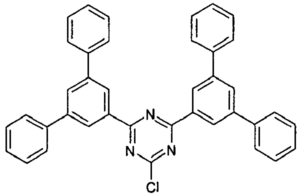

3.93 g Magnesium (162 mmol) werden in einem 500 mL Vierhalskolben vorgelegt, und eine Lösung aus 50 g 5'-Brom-[1,1';3',1"]terphenyl (162 mmol) in 150 ml THF wird langsam zugetropft. Die Reaktionsmischung wird 1.5 h zum Sieden erhitzt und anschließend auf Raumtemperatur abgekühlt. In einem zweiten Kolben wird Cyanchlorid (13 g, 70 mmol) in 150 ml THF vorgelegt und auf 0 °C gekühlt. Bei dieser Temperatur wird das abgekühlte Grignard-Reagenz zugetropft und 12 h bei Raumtemperatur gerührt. Nach dieser Zeit wird die Reaktionsmischung mit 150 ml HCl versetzt und die wässrige Phase dreimal mit Dichlormethan extrahiert. Die vereinigten organischen Phasen werden mit Wasser gewaschen, über Na₂SO₄ getrocknet und eingeengt. Der Rückstand wird aus EtOH umkristallisiert. Die Ausbeute beträgt 27.8 g (49 mol, 70%).

### Beispiel 5: 2-Chlor-4,6-bis-(3-([3,1';5,1"]terphen-1-yl)-phen-1-yl)-1,3,5-triazin

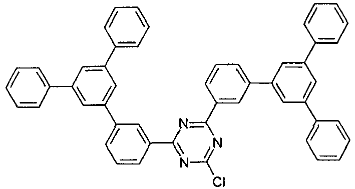

2.0 g Magnesium (81 mmol) werden in einem 500 mL Vierhalskolben vorgelegt, und eine Lösung von 31.2 g 5'-(3-Bromphenyl)-[1,1';3',1"]terphenyl (81 mmol) in 100 ml THF wird langsam zugetropft. Die Reaktionsmischung wird 1.5 h zum Sieden erhitzt und anschließend auf Raumtemperatur abgekühlt. In einem zweiten Kolben wird Cyanchlorid (6.4 g, 35 mmol) in 50 ml THF vorgelegt und auf 0 °C gekühlt. Bei dieser Temperatur wird das abgekühlte Grignard-Reagenz zugetropft und 12 h bei Raumtemperatur gerührt. Nach dieser Zeit wird die Reaktionsmischung mit 150 mL HCl versetzt, die wässrige Phase dreimal mit Dichlormethan extrahiert. Die vereinigten organischen Phasen werden mit Wasser gewaschen und über Na₂SO₄ getrocknet und eingeengt. Der Rückstand wird aus Toluol umkristallisiert. Die Ausbeute beträgt 6.8 g (9,4 mmol, 28%).

### Beispiel 6: 3-[3-(4,6-Diphenyl-[1,3,5]triazin-2-yl)-phenyl]-9-phenyl-9H-carbazol

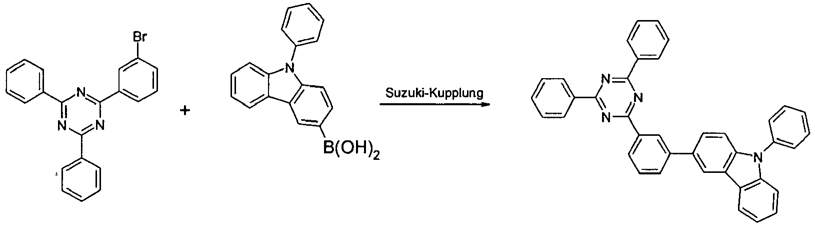

28.2 g (110.0 mmol) (9-Phenyl-9H-carbazol-3-yl)-boronsäure, 42.6 g (110.0 mmol) 2-(3-Brom-phenyl)-4,6-diphenyl-[1.3.5]triazin und 44.6 g (210.0 mmol) Trikaliumphosphat werden in 500 mL Toulol, 500 mL Dioxan und 500 mL Wasser suspendiert. Zu dieser Suspension werden 913 mg (3.0 mmol) Tri-o-tolylphosphin und dann 112 mg (0.5 mmol) Palladium(II)-acetat gegeben, und die Reaktionsmischung wird 16 h unter Rückfluss erhitzt. Nach Erkalten wird die organische Phase abgetrennt, über Kieselgel filtriert, dreimal mit 200 mL Wasser gewaschen und anschließend zur Trockene eingeengt. Der Rückstand wird aus Toluol und aus Dichlormethan / *iso*-Propanol umkristallisiert und abschließend im Hochvakuum (p = 5 x 10⁻⁷ mbar) sublimiert. Die Reinheit beträgt 99.9 % (HPLC). Die Ausbeute beträgt 52 g (94 mmol), entsprechend 86 % der Theorie.

Analog werden folgende Verbindungen erhalten:

| | **Edukt 1** | **Edukt 2** | **Produkt** | **Ausbeute** |
|---|---|---|---|---|
| 6a | 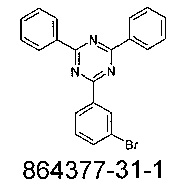 | 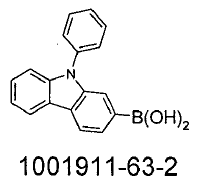 | 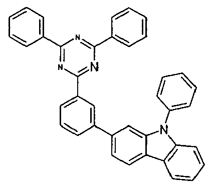 | 79% |
| 6b | 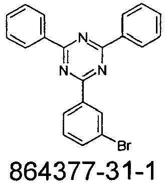 | 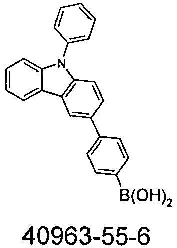 | 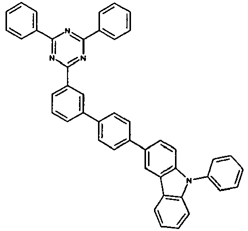 | 81% |
| 6c | 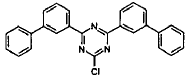 | 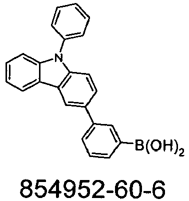 | 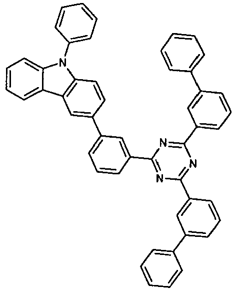 | 83% |
| 6d | 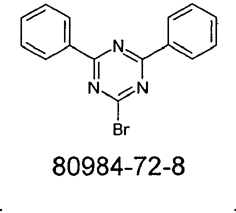 | 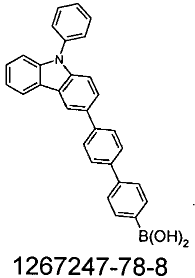 | 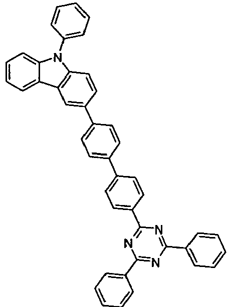 | 77% |
| 6e | 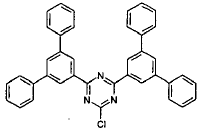 | 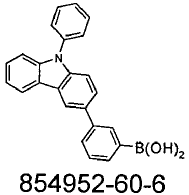 | 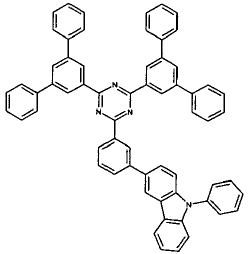 | 88% |
| 6f | 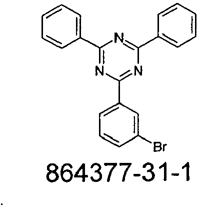 | 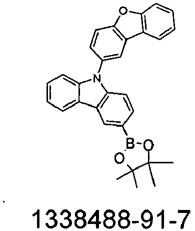 | 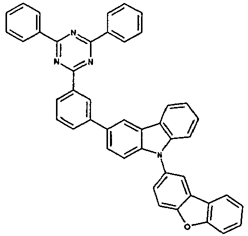 | 76% |
| 6g | 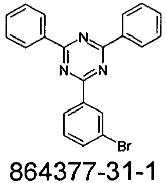 | 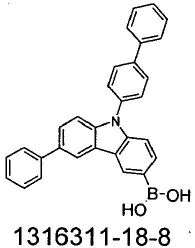 | 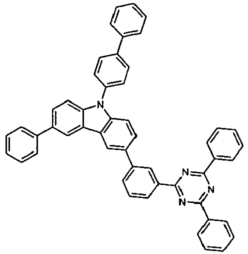 | 73% |
| 6h | 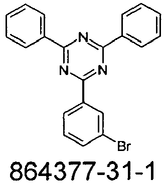 | 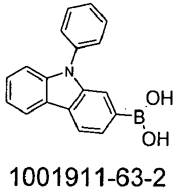 | 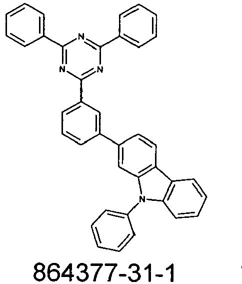 | 88% |
| 6i | 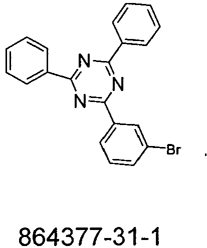 | 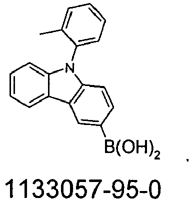 | 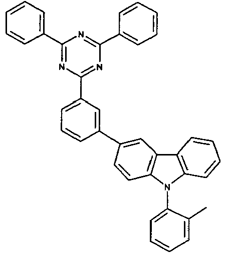 | 76% |
| 6j | 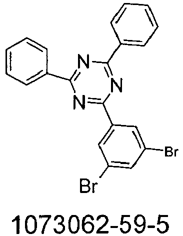 | 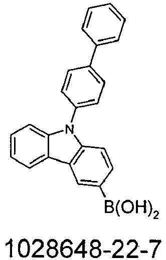 | 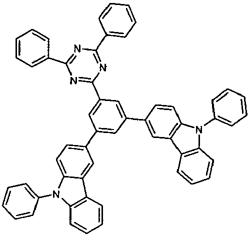 | 72% |
| 6k | 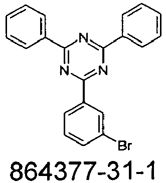 | 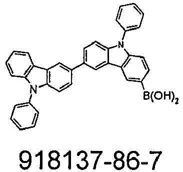 | 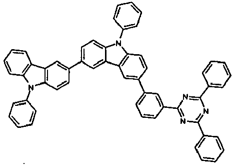 | 89% |
| 6l | 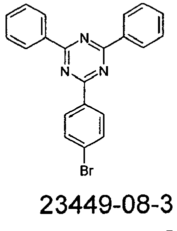 | 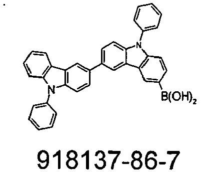 | 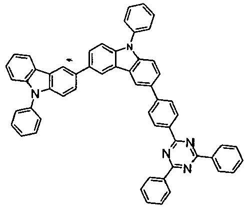 | 77% |
| 6m | 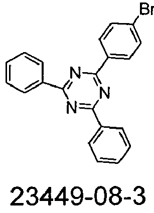 | 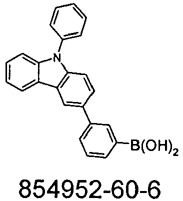 | 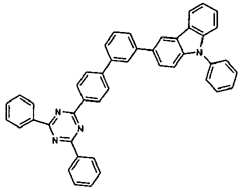 | 85% |
| 6n | 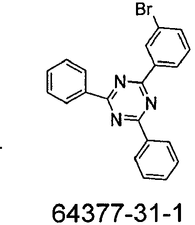 | 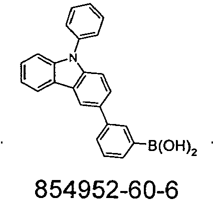 | 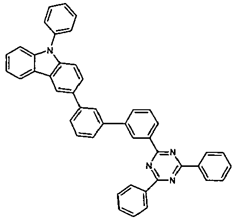 | 76% |
| 6o | 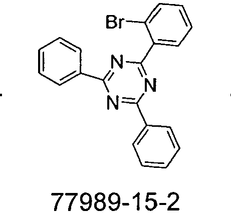 | 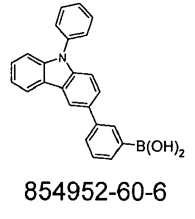 | 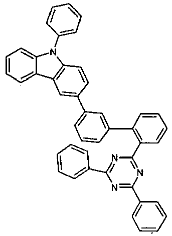 | 68% |
| 6p | 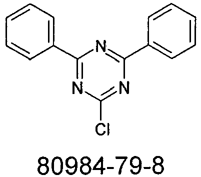 | 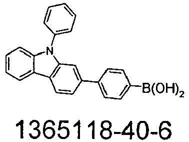 | 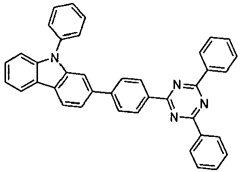 | 78% |
| 6q | 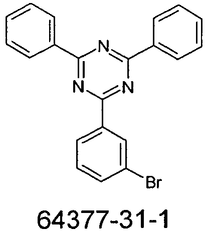 | 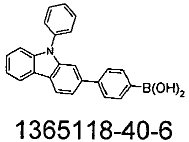 | 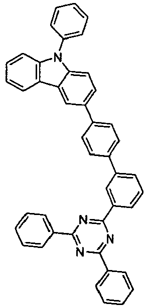 | 69% |
| 6r | 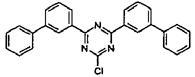 | 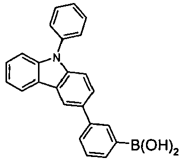 | 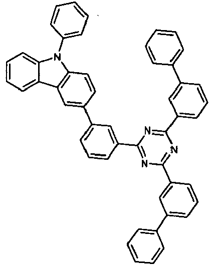 | 84% |
| 6s | 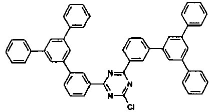 | 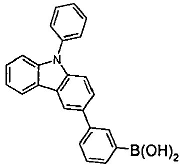 | 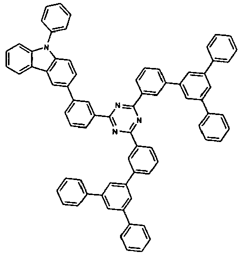 | 80% |
| 6t | 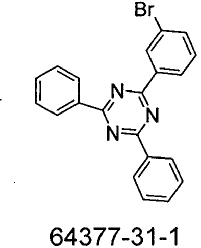 | 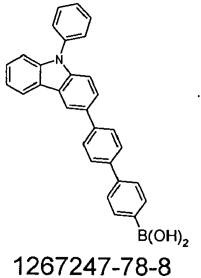 | 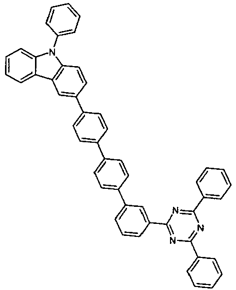 | 79% |
| 6u | 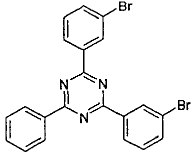 | 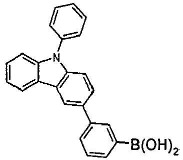 | 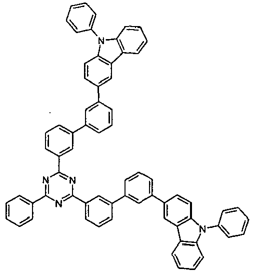 | 69% |

### Beispiel 7: 3-(5-Brom-biphenyl-3-yl)-9-phenyl-9H-carbazol

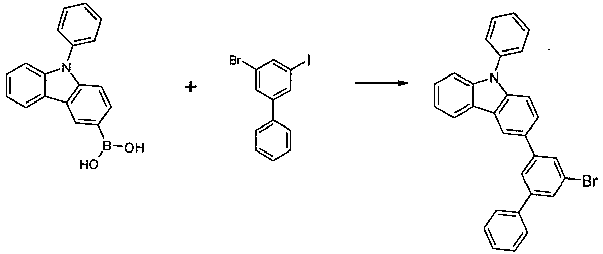

15.5 g (43.3 mmol) 3-Brom-5-iod-biphenyl und 13.7 g (48 mmol) (9-Phenyl-9H-carbazol-3-yl)-boronsäure werden in 80 mL Toluol gelöst und entgast. Es wird mit 281 mL einer entgasten 2M K₂CO₃-Lösung und mit 2.5 g (2.2 mmol) Pd(OAc)₂ versetzt. Die Reaktionsmischung wird anschließend bei 80 °C für 48 h unter Schutzgasatmosphäre gerührt. Die abgekühlte Lösung wird mit Toluol verdünnt, mehrmals mit Wasser gewaschen, getrocknet und eingeengt. Das Produkt wird via Säulenchromatographie an Kieselgel mit Toluol/Heptan (1:2) gereinigt. Die Reinheit beträgt 98 %. Ausbeute: 17.6 g (37 mmol, 78 %) der Theorie.

Analog werden folgende Verbindungen erhalten:

| | **Edukt 1** | **Edukt 2** | **Produkt** | **Ausbeute** |
|---|---|---|---|---|
| 7a | 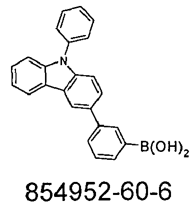 | 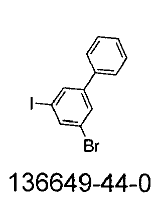 | 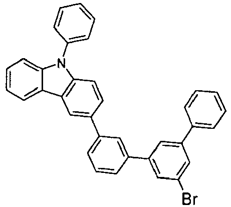 | 70% |
| 7b | 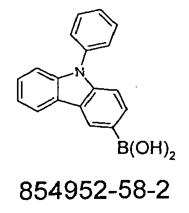 | 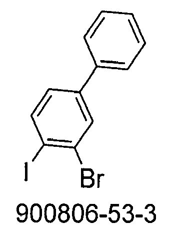 | 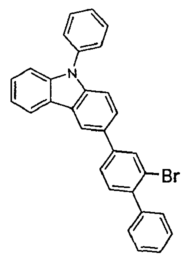 | 69% |
| 7c | 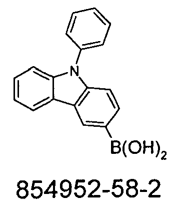 | 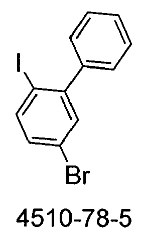 | 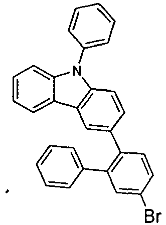 | 68% |
| 7d | 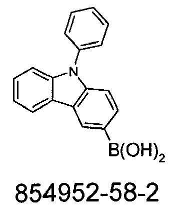 | 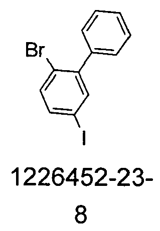 | 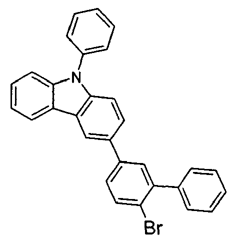 | 83% |

### Beispiel 8: 3-(5-Boronsäure-biphenyl-3-yl)-9-phenyl-9H-carbazole

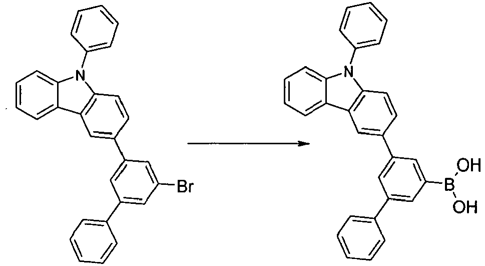

Eine auf-78 °C gekühlte Lösung von 128 g (270 mmol) 3-(5-Brombiphenyl-3-yl)-9-phenyl-9-H-carbazol in 1500 ml Diethylether wird tropfenweise mit 110 ml (276 mmol) n-Buthyllithium (2.5 M in Hexan) versetzt. Die Reaktionsmischung wird 30 min. bei -78 °C gerührt. Man lässt auf Raumtemperatur kommen, kühlt erneut auf -78 °C und versetzt dann schnell mit einer Mischung von 40 ml (351 mmol) Trimethylborat in 50 ml Diethylether. Nach Erwärmen auf -10 °C wird mit 135 ml 2 N Salzsäure hydrolysiert. Die organische Phase wird abgetrennt, mit Wasser gewaschen, über Natriumsulfat getrocknet und zur Trockene eingeengt. Der Rückstand wird in 300 ml n-Heptan aufgenommen, der farblose Feststoff wird abgesaugt, mit n-Heptan gewaschen und im Vakuum getrocknet. Ausbeute: 112 g (256 mmol), 95 % d. Th..

Analog werden folgende Verbindungen erhalten:

| | **Edukt 1** | **Produkt** | **Ausbeute** |
|---|---|---|---|
| 8a | 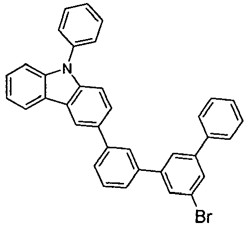 | 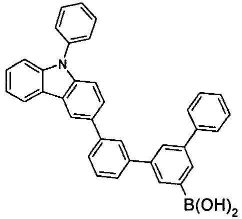 | 64% |
| 8b | 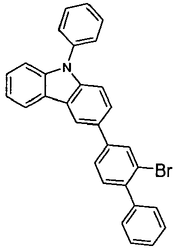 | 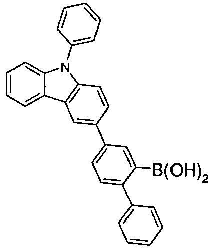 | 60% |
| 8c | 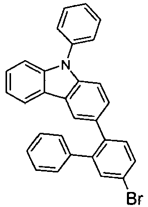 | 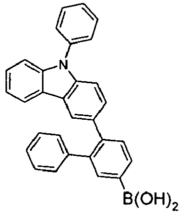 | 63% |
| 8d | 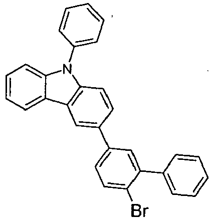 | 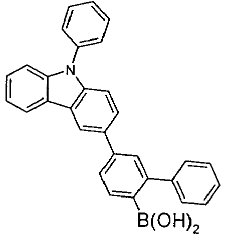 | 69% |
| 8e | 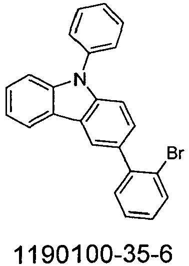 | 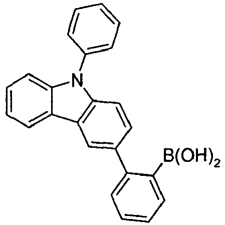 | 59% |
| 8f | 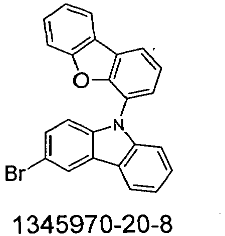 | 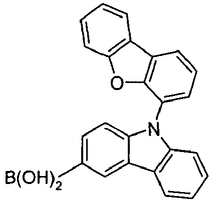 | 83% |
| 8g | 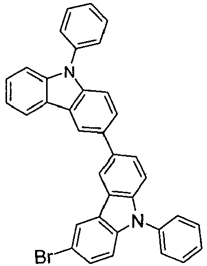 | 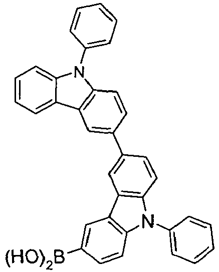 | 73% |

### Beispiel 9: 3-[3"-(4,6-Diphenyl-[1,3,5]triazin-2-yl)-[1,1';3',1"]terphenyl-5'-yl]-9-phenyl-9H-carbazol

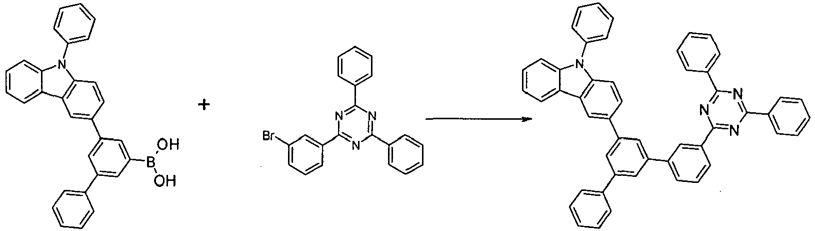

Eine gut gerührte Suspension von 15.5 g (40 mmol) 2-(3-Bromo-phenyl)-4,6-diphenyl-[1,3,5]triazine, 17,5 g (40 mmol) 3-(5-Boronsäure-biphenyl-3-yl)-9-phenyl-9H-carbazole und 63.9 g (127 mmol) Na₂CO₃ in 500 ml DMF wird mit 2.47 g (8.1 mmol) Tetrakis-triphenylphosphinopalladium(0) versetzt und anschließend 16 h unter Rückfluss erhitzt. Nach Erkalten wird der ausgefallene Feststoff abgesaugt, dreimal mit 50 ml Toluol, dreimal mit 50 ml Ethanol:Wasser (1:1, v:v) und dreimal mit 100 ml Ethanol gewaschen und dreimal aus DMF (ca. 15 ml/g) umkristallisiert. und abschließend im Hochvakuum (p = 5 x 10⁻⁷ mbar) sublimiert. Ausbeute 27 g (38 mmol), 85.0 % d. Th.; Reinheit 99.9% (HPLC)

Analog werden folgende Verbindungen erhalten:

| | **Edukt 1** | **Edukt 2** | **Produkt** | **Ausbeute** |
|---|---|---|---|---|
| 9a | 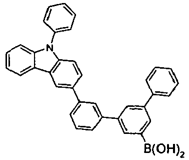 | 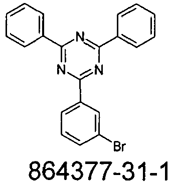 | 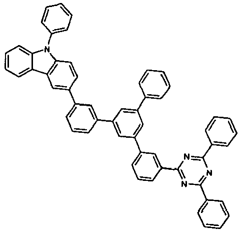 | 86% |
| 9b | 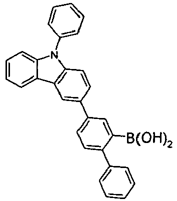 | 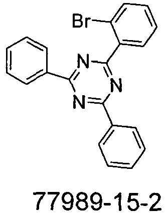 | 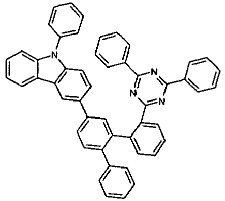 | 67% |
| 9c | 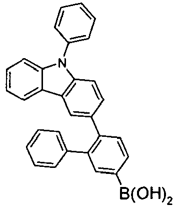 | 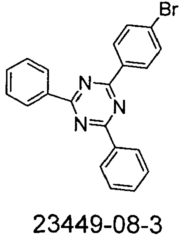 | 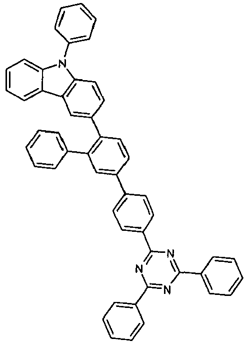 | 69% |
| 9d | 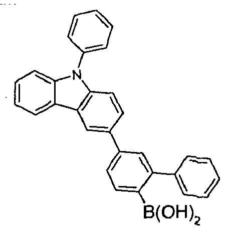 | 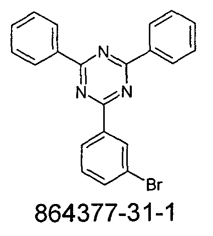 | 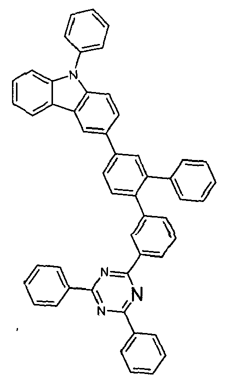 | 76% |
| 9e | 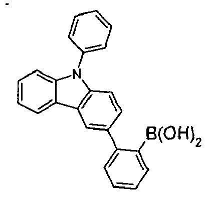 | 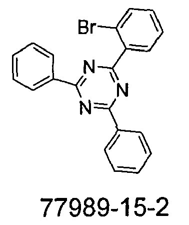 | 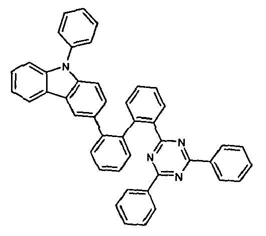 | 82% |
| 9f | 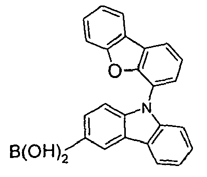 | 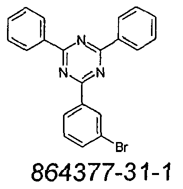 | 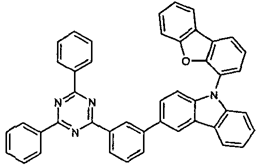 | 80% |
| 9g | 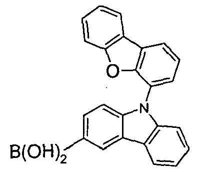 | 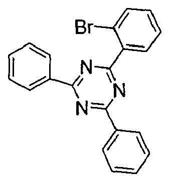 | 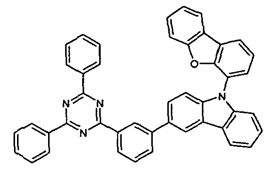 | 64% |
| 9h | 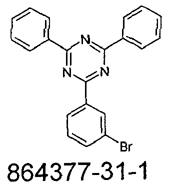 | 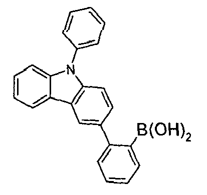 | 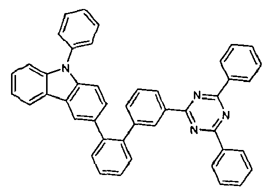 | 73% |
| 9i | 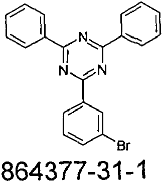 | 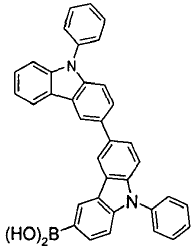 | 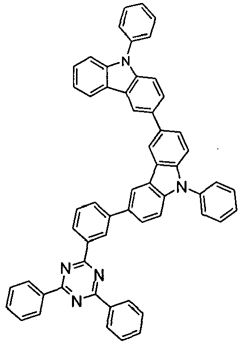 | 71% |

### Beispiel: Herstellung der OLEDs

Die Herstellung von erfindungsgemäßen OLEDs sowie OLEDs nach dem Stand der Technik erfolgt nach einem allgemeinen Verfahren gemäß WO 04/058911, das auf die hier beschriebenen Gegebenheiten (Schichtdickenvariation, Materialien) angepasst wird.

In den folgenden Beispielen V1 bis E17 (siehe 1 und 2) werden die Daten verschiedener OLEDs vorgestellt. Glasplättchen, die mit strukturiertem ITO (Indium Zinn Oxid) der Dicke 50 nm beschichtet sind werden zur verbesserten Prozessierung mit 20 nm PEDOT:PSS beschichtet (Poly(3,4-ethylendioxythiophen)poly(styrolsulfonat), bezogen als CLEVIOS™ P VP Al 4083 von Heraeus Precious Metals GmbH Deutschland, aus wässriger Lösung aufgeschleudert). Diese beschichteten Glasplättchen bilden die Substrate, auf welche die OLEDs aufgebracht werden. Die OLEDs haben prinzipiell folgenden Schichtaufbau: Substrat / Lochtransportschicht (HTL) / Zwischenschicht (IL) / Elektronenblockerschicht (EBL) / Emissionsschicht (EML) / optionale Lochblockierschicht (HBL) / Elektronentransportschicht (ETL) / optionale Elektroneninjektionsschicht (EIL) und abschließend eine Kathode. Die Kathode wird durch eine 100 nm dicke Aluminiumschicht gebildet. Der genaue Aufbau der OLEDs ist Tabelle 1 zu entnehmen. Die zur Herstellung der OLEDs benötigten Materialien sind in Tabelle 3 gezeigt. Eine Bezeichnung wie "6a" bezieht sich hierbei auf das im oben beschriebenen Beispiel 6a genannte Material. Dies gilt analog auch für alle anderen erfindungsgemäßen Verbindungen.

Alle Materialien werden in einer Vakuumkammer thermisch aufgedampft. Dabei besteht die Emissionsschicht immer aus mindestens einem Matrixmaterial (Hostmaterial, Wirtsmaterial) und einem emittierenden Dotierstoff (Dotand, Emitter), der dem Matrixmaterial bzw. den Matrixmaterialien durch Coverdampfung in einem bestimmten Volumenanteil beigemischt wird. Eine Angabe wie H1:VCbz1:TEG1 (55%:35%:10%) bedeutet hierbei, dass das Material H1 in einem Volumenanteil von 55%, VCbz1 in einem Anteil von 35% und TEG1 in einem Anteil von 10% in der Schicht vorliegt. Analog kann auch die Elektronentransportschicht aus einer Mischung von zwei Materialien bestehen.

Die OLEDs werden standardmäßig charakterisiert. Hierfür werden die Elektrolumineszenzspektren, die Stromeffizienz (gemessen in cd/A), die Leistungseffizienz (gemessen in Im/W) und die externe Quanteneffizienz (EQE, gemessen in Prozent) in Abhängigkeit der Leuchtdichte, berechnet aus Strom-Spannungs-Leuchtdichte-Kennlinien (IUL-Kennlinien) unter Annahme einer lambertschen Abstrahlcharakteristik sowie die Lebensdauer bestimmt. Die Elektrolumineszenzspektren werden bei einer Leuchtdichte von 1000 cd/m² bestimmt und daraus die CIE 1931 x und y Farbkoordinaten berechnet. Die Angabe U1000 in Tabelle 2 bezeichnet die Spannung, die für eine Leuchtdichte von 1000 cd/m² benötigt wird. SE1000 und LE1000 bezeichnen die Strom- bzw. Leistungseffizienz, die bei 1000 cd/m² erreicht werden. EQE1000 schließlich bezeichnet die externe Quanteneffizienz bei einer Betriebsleuchtdichte von 1000 cd/m². Als Lebensdauer LD wird die Zeit definiert, nach der die Leuchtdichte bei Betrieb mit konstantem Strom von der Startleuchtdichte L0 auf einen gewissen Anteil L1 absinkt. Eine Angabe von L0 = 10000 cd/m² und L1 = 80% in Tabelle X2 bedeutet, dass die in Spalte LD angegebene Lebensdauer der Zeit entspricht, nach der die Anfangsleuchtdichte von 10000 cd/m² auf 8000 cd/m² absinkt.

Die Daten der verschiedenen OLEDs sind in Tabelle 2 zusammengefasst. Die Beispiel V1-V6 sind OLEDs enthaltend Materialien gemäß dem Stand der Technik, die Beispiele E1-E17 zeigen Daten von OLEDs mit erfindungsgemäßen Materialien.

Im Folgenden werden einige der Beispiele näher erläutert, um die Vorteile der erfindungsgemäßen Verbindungen zu verdeutlichen. Es sei jedoch darauf hingewiesen, dass dies nur eine Auswahl der in Tabelle 2 gezeigten Daten darstellt. Wie sich der Tabelle entnehmen lässt, werden auch bei Verwendung der nicht näher ausgeführten erfindungsgemäßen Verbindungen Verbesserungen gegenüber dem Stand der Technik erzielt, teilweise in allen Parametern, in manchen Fällen ist aber nur eine Verbesserung von Effizienz oder Spannung oder Lebensdauer zu beobachten. Allerdings stellt bereits die Verbesserung einer der genannten Paramter einen signifikanten Fortschritt dar, weil verschiedene Anwendungen die Optimierung hinsichtlich unterschiedlicher Parameter erfordern.

### Verwendung von erfindungsgemäßen Verbindungen als Matrixmaterialien in phosphoreszierenden OLEDs

In Kombination mit dem grün emittierenden Dotanden TEG1 zeigen erfindungsgemäße Materialien deutliche Verbesserungen gegenüber dem Stand der Technik. Man erhält eine um bis zu 15% verbesserte Leistungseffizienz (Beispiele V1 und E3) und 40% bessere Lebensdauer (Beispiele V2 und E3).

Verwendet man zwei Materialien als Mischung mit dem Dotanden TEG1 in der EML, so erhält man mit dem erfindungsgemäßen Material 6n in Kombination mit VCbz1 eine um etwa 30% verbesserte Lebensdauer sowie etwa 20% höhere Leistungseffizienz gegenüber Verwendung von H2 mit VCbz1 (Beispiele V4 und E10).

Ähnlich gute Verbesserungen erhält man auch bei Einsatz des rot emittierenden Dotanden TER1 (Beispiele V6 und E17).

Die erfindungsgemäßen Materialien ergeben bei Einsatz als Matrixmaterialien in phosphoreszierenden OLEDs somit wesentliche Verbesserungen gegenüber dem Stand der Technik.

### Verwendung von erfindungsgemäßen Verbindungen als Elektronentransportmaterialien

Bei Verwendung der erfindungsgemäßen Verbindung 6n als Elektronentransportmaterial erhält man wesentlich geringere Spannung und bessere Effizienz als mit der Substanz H3 gemäß dem Stand der Technik (Beispiele V5 und E16).

**Tabelle 1: Aufbau der OLEDs**

| Bsp. | HTL Dicke | IL Dicke | EBL Dicke | EML Dicke | HBL Dicke | ETL Dicke | EIL Dicke |
|---|---|---|---|---|---|---|---|
| V1 | SpA1 70nm | HATCN 5nm | SpMA1 90nm | H1:TEG1 (90%:10%) 30nm | --- | ST1:LiQ (50%:50%) 40nm | --- |
| V2 | SpA1 70nm | HATCN 5nm | SpMA1 90nm | H2:TEG1 (90%:10%) 30nm | --- | ST1:LiQ (50%:50%) 40nm | --- |
| V3 | SpA1 70nm | HATCN 5nm | SpMA1 90nm | H3:TEG1 (90%:10%) 30nm | --- | ST1:LiQ (50%:50%) 40nm | --- |
| V4 | SpA1 70nm | HATCN 5nm | SpMA1 90nm | H2:VCbz1:TEG1 (45%:45%:10%) 30nm | IC1 10nm | ST1:LiQ (50%:50%) 30nm | --- |
| V5 | SpA1 70nm | HATCN 5nm | SpMA1 90nm | IC1:TEG1 (90%:10%) 30nm | --- | H3 40nm | LiQ 3nm |
| V6 | SpA1 90nm | HATCN 5nm | SpMA1 130nm | H1:TER1 (92%:8%) 40nm | --- | ST1:LiQ(50%:50%) 40nm | --- |
| E1 | SpA1 70nm | HATCN 5nm | SpMA1 90nm | 6:TEG1 (90%:10%) 30nm | --- | ST1:LiQ (50%:50%) 40nm | --- |
| E2 | SpA1 70nm | HATCN 5nm | SpMA1 90nm | 6a:TEG1 (90%:10%) 30nm | --- | ST1:LiQ(50%:50%) 40nm | --- |
| E3 | SpA1 70nm | HATCN 5nm | SpMA1 90nm | 6b:TEG1 (90%:10%) 30nm | --- | ST1:LiQ (50%:50%) 40nm | --- |
| E4 | SpA1 70nm | HATCN 5nm | SpMA1 90nm | 6c:TEG1 (90%:10%) 30nm | --- | ST1:LiQ (50%:50%) 40nm | --- |
| E5 | SpA1 70nm | HATCN 5nm | SpMA1 90nm | 6f:TEG1 (90%:10%) 30nm | --- | ST1:LiQ (50%:50%) 40nm | --- |
| E6 | SpA1 70nm | HATCN 5nm | SpMA1 90nm | 6h:TEG1 (90%:10%) 30nm | --- | ST1:LiQ (50%:50%) 40nm | --- |
| E7 | SpA1 70nm | HATCN 5nm | SpMA1 90nm | 6j:TEG1 (90%:10%) 30nm | --- | ST1:LiQ (50%:50%) 40nm | --- |
| E8 | SpA1 70nm | HATCN 5nm | SpMA1 90nm | 6k:TEG1 (90%:10%) 30nm | --- | ST1:LiQ (50%:50%) 40nm | --- |
| E9 | SpA1 70nm | HATCN 5nm | SpMA1 90nm | 6n:TEG1 (90%:10%) 30nm | --- | ST1:LiQ (50%:50%) 40nm | --- |
| E10 | SpA1 70nm | HATCN 5nm | SpMA1 90nm | 6n:VCbz1:TEG1 (45%:45%:10%) 30nm | IC1 10nm | ST1:LiQ (50%:50%) 30nm | --- |
| E11 | SpA1 70nm | HATCN 5nm | SpMA1 90nm | 6o:TEG1 (90%:10%) 30nm | --- | ST1:LiQ (50%:50%) 40nm | --- |
| E12 | SpA1 70nm | HATCN 5nm | SpMA1 90nm | 6p:TEG1 (90%:10%) 30nm | --- | ST1:LiQ (50%:50%) 40nm | --- |
| E13 | SpA1 70nm | HATCN 5nm | SpMA1 90nm | 9:TEG1 (90%:10%) 30nm | --- | ST1:LiQ (50%:50%) 40nm | --- |
| E14 | SpA1 70nm | HATCN 5nm | SpMA1 90nm | 9f:TEG1 (90%:10%) 30nm | --- | ST1:LiQ (50%:50%) 40nm | --- |
| E15 | SpA1 70nm | HATCN 5nm | SpMA1 90nm | 9h:TEG1 (90%:10%) 30nm | --- | ST1:LiQ (50%:50%) 40nm | --- |
| E16 | SpA1 70nm | HATCN 5nm | SpMA1 90nm | IC1:TEG1 (90%:10%) 30nm | --- | 6n 40nm | LiQ 3nm |
| E17 | SpA1 90nm | HATCN 5nm | SpMA1 130nm | 6n:TER1 (92%:8%) 40nm | --- | ST1:LiQ (50%:50%) 40nm | --- |

**Tabelle 2: Daten der OLEDs**

| Bsp. | U1000 (V) | SE1000 (cd/A) | LE1000 (lm/W) | EQE 1000 | CIE x/y bei 1000 cd/m² | L0 | L1 % | LD (h) |
|---|---|---|---|---|---|---|---|---|
| V1 | 4.0 | 55 | 43 | 15.3% | 0.33/0.62 | 10000 cd/m² | 80 | 65 |
| V2 | 4.1 | 44 | 34 | 12.3% | 0.32/0.62 | 10000 cd/m² | 80 | 110 |
| V3 | 4.9 | 56 | 36 | 15.6% | 0.33/0.62 | 10000 cd/m² | 80 | 105 |
| V4 | 4.0 | 50 | 39 | 13.8% | 0.33/0.62 | 10000 cd/m² | 80 | 240 |
| V5 | 4.4 | 53 | 38 | 14.7% | 0.33/0.62 | 10000 cd/m² | 80 | 90 |
| V6 | 4.6 | 9.3 | 6.4 | 9.8% | 0.67/0.33 | 4000 cd/m² | 80 | 290 |
| E1 | 4.2 | 56 | 42 | 15.5% | 0.32/0.62 | 10000 cd/m² | 80 | 85 |
| E2 | 4.2 | 55 | 41 | 15.3% | 0.33/0.63 | 10000 cd/m² | 80 | 115 |
| E3 | 3.7 | 59 | 50 | 16.5% | 0.32/0.62 | 10000 cd/m² | 80 | 155 |
| E4 | 4.4 | 54 | 39 | 15.0% | 0.33/0.62 | 10000 cd/m² | 80 | 130 |
| E5 | 4.1 | 58 | 44 | 16.1% | 0.33/0.62 | 10000 cd/m² | 80 | 120 |
| E6 | 4.3 | 51 | 38 | 14.3% | 0.33/0.62 | 10000 cd/m² | 80 | 110 |
| E7 | 4.0 | 57 | 45 | 15.9% | 0.33/0.62 | 10000 cd/m² | 80 | 135 |
| E8 | 4.8 | 53 | 35 | 14.8% | 0.33/0.63 | 10000 cd/m² | 80 | 90 |
| E9 | 4.0 | 60 | 47 | 16.8% | 0.33/0.62 | 10000 cd/m² | 80 | 140 |
| E10 | 3.8 | 57 | 47 | 15.8% | 0.33/0.62 | 10000 cd/m² | 80 | 305 |
| E11 | 4.2 | 59 | 44 | 16.4% | 0.33/0.62 | 10000 cd/m² | 80 | 140 |
| E12 | 4.1 | 57 | 44 | 15.9% | 0.32/0.63 | 10000 cd/m² | 80 | 110 |
| E13 | 4.1 | 59 | 45 | 16.3% | 0.33/0.62 | 10000 cd/m² | 80 | 130 |
| E14 | 3.7 | 55 | 46 | 15.2% | 0.33/0.62 | 10000 cd/m² | 80 | 105 |
| E15 | 4.2 | 60 | 45 | 16.6% | 0.33/0.62 | 10000 cd/m² | 80 | 135 |
| E16 | 3.2 | 59 | 58 | 16.5% | 0.33/0.62 | 10000 cd/m² | 80 | 110 |
| E17 | 4.5 | 10.8 | 7.6 | 11.7% | 0.67/0.33 | 4000 cd/m² | 80 | 360 |

**Tabelle 3: Strukturformeln der Materialien für die OLEDs**

| | |
|---|---|
| 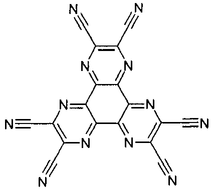 | 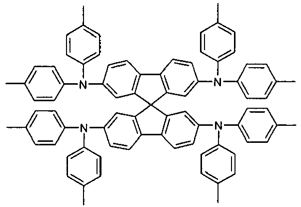 |
| HATCN | SpA1 |
| 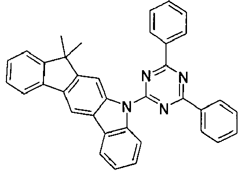 | 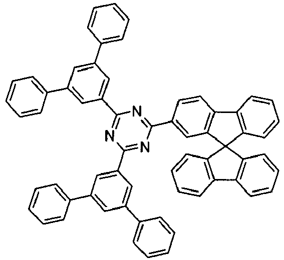 |
| IC1 | ST1 |
| 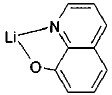 | 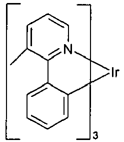 |
| LiQ | TEG1 |
| 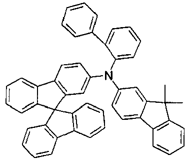 | 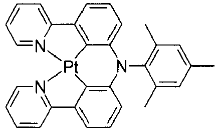 |
| SpMA1 | TER1 |
| 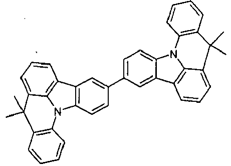 | 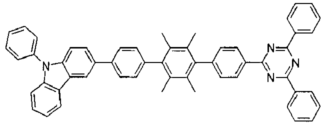 |
| VCbz1 | H1 (Stand der Technik gemäß JP 2004/071500) |
| 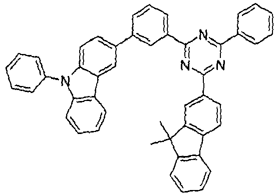 | 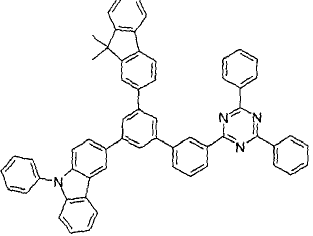 |
| H2 (Stand der Technik gemäß WO 2011/046182) | H3 (Stand der Technik) (Stand der technik gemäß WO 2011/046182) |

## Patentansprüche

1. Verbindung gemäß Formel (1), 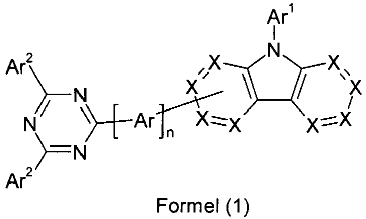 wobei für die verwendeten Symbole und Indizes gilt:
X ist bei jedem Auftreten gleich oder verschieden CR¹ oder N; bzw. X ist C, wenn an dieser Position die Gruppe Ar gebunden ist;
Ar ist bei jedem Auftreten gleich oder verschieden eine Phenylengruppe, welche durch einen oder mehrere Reste R substituiert sein kann;
Ar¹ ist ein aromatisches Ringsystem mit 6 bis 24 aromatischen Ringatomen, welches keine kondensierten Arylgruppen mit mehr als 10 aromatischen Ringatomen aufweist und welches durch einen oder mehrere Reste R¹ substituiert sein kann, oder ist eine Dibenzofuran- oder Dibenzothiophengruppe, welche jeweils durch einen oder mehrere Reste R¹ substituiert sein kann;
Ar² ist bei jedem Auftreten gleich oder verschieden eine Arylgruppe, eine Biarylgruppe, eine Triarylgruppe oder Quaterarylgruppe, wobei jede einzelne der Arylgruppen in den vorstehend genannten Gruppen 6 bis 10 aromatische Ringatome hat und mit einem oder mehreren Resten R¹ substituiert sein kann, oder ist eine Dibenzofuran- oder Dibenzothiophengruppe, welche jeweils durch einen oder mehrere Reste R¹ substituiert sein kann;
R ist bei jedem Auftreten gleich oder verschieden H, D, F, CN, eine Arylgruppe, eine Biarylgruppe, eine Triarylgruppe oder Quaterarylgruppe, wobei jede einzelne der Arylgruppen in den vorstehend genannten Gruppen 6 bis 10 aromatische Ringatome hat und mit einem oder mehreren Resten R¹ substituiert sein kann, oder eine über ein Kohlenstoffatom verknüpfte Carbazolgruppe, welche auch mit einem oder mehreren Reste R¹ substituiert sein kann;
R¹ ist bei jedem Auftreten gleich oder verschieden H, D, F, CN, eine geradkettige Alkyl- oder Alkoxygruppe mit 1 bis 20 C-Atomen oder eine verzweigte oder cyclische Alkyl- oder Alkoxygruppe mit 3 bis 20 C-Atomen, wobei eine oder mehrere nicht benachbarte CH₂-Gruppen durch R²C=CR², C≡C oder O ersetzt sein können und wobei ein oder mehrere H-Atome durch D oder F ersetzt sein können, oder eine Arylgruppe mit 6 bis 10 C-Atomen, die durch einen oder mehrere Reste R² substituiert sein kann, oder ist eine Dibenzofuran- oder Dibenzothiophengruppe, welche jeweils durch einen oder mehrere Reste R² substituiert sein kann, oder eine über ein Kohlenstoffatom verknüpfte Carbazolgruppe, welche auch mit einem oder mehreren Reste R² substituiert sein kann, oder eine Aralkylgruppe mit 6 bis 10 aromatischen Ringatomen, die durch einen oder mehrere Reste R² substituiert sein kann; dabei können zwei benachbarte Substituenten R¹ zusammen mit den Atomen, an die sie gebunden sind, auch miteinander ein mono- oder polycyclisches, aliphatisches Ringsystem bilden;
R² ist bei jedem Auftreten gleich oder verschieden H, D oder ein aliphatischer Kohlenwasserstoffrest mit 1 bis 20 C-Atomen oder eine Arylgruppe mit 6 bis 10 Ringatomen;
n ist 1, 2, 3 oder 4, bevorzugt 1, 2 oder 3.

2. Verbindung nach Anspruch 1, **dadurch gekennzeichnet, dass** maximal eine Gruppe X pro Cyclus für N steht und die verbleibenden Gruppen X gleich oder verschieden bei jedem Auftreten für CR¹ stehen.

3. Verbindung nach Anspruch 1 gemäß Formel (2), 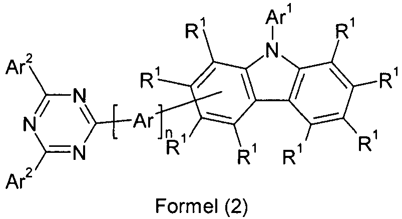 wobei die verwendeten Symbole und Indizes die in Anspruch 1 genannten Bedeutungen aufweisen und der Rest R¹ nicht vorhanden ist, wenn an dieser Position die Gruppe Ar gebunden ist.

4. Verbindung nach einem oder mehreren der Ansprüche 1 bis 3, ausgewählt aus den Verbindungen der Formeln (2a), (2b) und (2c), 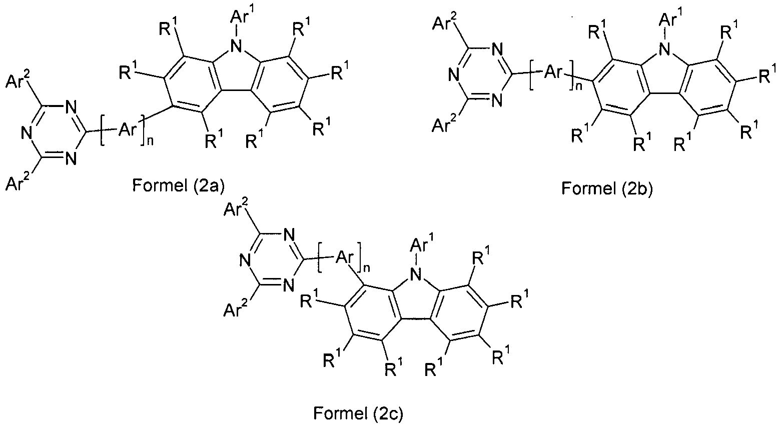 wobei die verwendeten Symbole und Indizes die in Anspruch 1 genannten Bedeutungen aufweisen.

5. Verbindung nach einem oder mehreren der Ansprüche 1 bis 4, **dadurch gekennzeichnet, dass** n = 1, 2 oder 3 ist und Ar gleich oder verschieden bei jedem Auftreten ausgewählt ist aus den Formeln (3), (4) oder (5), 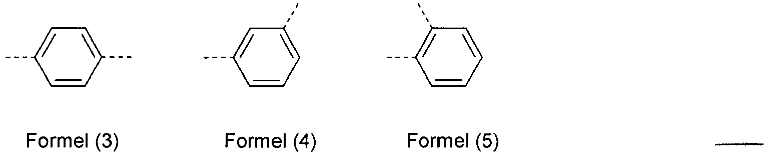 wobei die gestrichelte Bindung jeweils die Verknüpfung dieser Gruppen andeutet und jede dieser Gruppen durch einen oder mehrere Reste R substituiert sein kann.

6. Verbindung nach einem oder mehreren der Ansprüche 1 bis 5, **dadurch gekennzeichnet, dass** die Gruppe -(Ar)ₙ- ausgewählt ist aus den Formeln (6) bis (18), 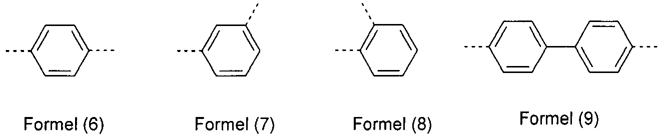 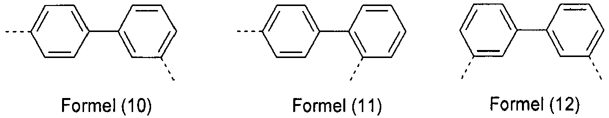 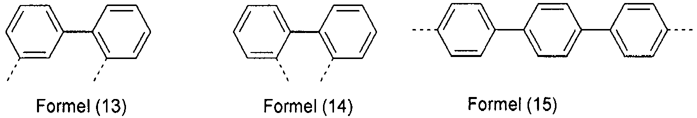 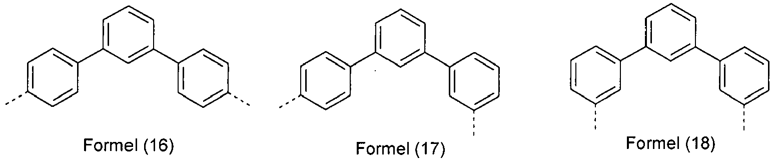 wobei die gestrichelte Bindung jeweils die Verknüpfung dieser Gruppen andeutet, jede dieser Gruppen auch durch einen oder mehrere Reste R substituiert sein kann und R und R¹ die in Anspruch 1 genannten Bedeutungen aufweisen.

7. Verbindung nach einem oder mehreren der Ansprüche 1 bis 6, **dadurch gekennzeichnet, dass** der Rest R gleich oder verschieden bei jedem Auftreten ausgewählt ist aus der Gruppe bestehend aus H, D, F, CN, Phenyl, Biphenyl oder Terphenyl, wobei jede der Arylgruppen in den vorstehend genannten Gruppen mit einem oder mehreren Resten R¹ substituiert sein kann und R¹ gleich oder verschieden bei jedem Auftreten für H oder eine Alkylgruppe mit 1 bis 5 C-Atomen steht; oder eine über ein Kohlenstoffatom verknüpfte Carbazolgruppe, welche mit einem oder mehreren Reste R¹ substituiert sein kann.

8. Verbindung nach einem oder mehreren der Ansprüche 1 bis 7, **dadurch gekennzeichnet, dass** Ar¹ ausgewählt ist aus der Gruppe bestehend aus Phenyl, Biphenyl, Terphenyl oder Quaterphenyl, welches jeweils mit einem oder mehreren Resten R¹ substituiert sein kann.

9. Verbindung nach einem oder mehreren der Ansprüche 1 bis 8, **dadurch gekennzeichnet, dass** Ar² gleich oder verschieden bei jedem Auftreten ausgewählt ist aus der Gruppe bestehend aus Phenyl, Biphenyl, Terphenyl oder Quaterphenyl, welches jeweils mit einem oder mehreren Resten R¹ substituiert sein kann.

10. Verbindung nach einem oder mehreren der Ansprüche 1 bis 9, **dadurch gekennzeichnet, dass** für die Symbole und Indizes gilt:
Ar steht gleich oder verschieden bei jedem Auftreten für eine Gruppe der oben aufgeführten Formel (3), (4) oder (5) gemäß Anspruch 5, wobei n = 1, 2 oder 3 ist;
R ist gleich oder verschieden bei jedem Auftreten ausgewählt aus der Gruppe bestehend aus H, D, F, CN, Phenyl, Biphenyl oder Terphenyl, wobei jede der Arylgruppen mit einem oder mehreren Resten R¹ substituiert sein kann und R¹ gleich oder verschieden bei jedem Auftreten für H oder eine Alkylgruppe mit 1 bis 5 C-Atomen steht; oder eine über ein Kohlenstoffatom verknüpfte Carbazolgruppe, welche auch mit einem oder mehreren Reste R¹ substituiert sein kann.
Ar¹ ist ausgewählt aus der Gruppe bestehend aus Phenyl, Biphenyl, Terphenyl oder Quaterphenyl, welches jeweils mit einem oder mehreren Resten R¹ substituiert sein kann;
Ar² ist gleich oder verschieden bei jedem Auftreten ausgewählt aus der Gruppe bestehend aus Phenyl, Biphenyl, Terphenyl oder Quaterphenyl, welches jeweils mit einem oder mehreren Resten R¹ substituiert sein kann;
R¹ ist bei jedem Auftreten gleich oder verschieden ausgewählt aus der Gruppe bestehend aus H, D, F, einer geradkettigen Alkylgruppe mit 1 bis 10 C-Atomen oder einer verzweigten oder cyclischen Alkylgruppe mit 3 bis 10 C-Atomen, wobei eine oder mehrere nicht benachbarte CH₂-Gruppen durch R²C=CR² ersetzt sein können und wobei ein oder mehrere H-Atome durch F ersetzt sein können, oder eine Arylgruppe mit 6 bis 10 C-Atomen, die durch einen oder mehrere Reste R² substituiert sein kann, oder ist eine Dibenzofuran- oder Dibenzothiophengruppe, welche jeweils durch einen oder mehrere Reste R² substituiert sein kann, oder eine über ein Kohlenstoffatom verknüpfte Carbazolgruppe, welche auch mit einem oder mehreren Reste R² substituiert sein kann.

11. Verfahren zur Herstellung einer Verbindung nach einem oder mehreren der Ansprüche 1 bis 10, **dadurch gekennzeichnet, dass** die Einheit (Ar²)₂Triazin-(Ar)ₙ durch eine Suzuki-Kupplung eingeführt wird.

12. Mischung enthaltend mindestens eine Verbindung nach einem oder mehreren der Ansprüche 1 bis 10 und mindestens eine weitere Verbindung, insbesondere ein fluoreszierender oder phosphoreszierender Dotand.

13. Formulierung, insbesondere eine Lösung, eine Suspension oder eine Miniemulsion, enthaltend mindestens eine Verbindung nach einem oder mehreren der Ansprüche 1 bis 10 oder eine Mischung nach Anspruch 12 und ein oder mehrere Lösemittel.

14. Verwendung einer Verbindung nach einem oder mehreren der Ansprüche 1 bis 10 oder einer Mischung nach Anspruch 12 in einer elektronischen Vorrichtung, insbesondere in einer organischen Elektrolumineszenzvorrichtung.

15. Elektronische Vorrichtung enthaltend mindestens eine Verbindung nach einem oder mehreren der Ansprüche 1 bis 10 oder mindestens eine Mischung nach Anspruch 12, bevorzugt ausgewählt aus der Gruppe bestehend aus organischen Elektrolumineszenzvorrichtungen, organischen integrierten Schaltungen, organischen Feld-Effekt-Transistoren, organischen Dünnfilmtransistoren, organischen lichtemittierenden Transistoren, organischen Solarzellen, organischen farbstoff-sensibilisierten Solarzellen, organischen optischen Detektoren, organischen Photorezeptoren, organischen Feld-Quench-Devices, lichtemittierenden elektrochemischen Zellen, organischen Laserdioden und "organic plasmon emitting devices".

16. Elektronische Vorrichtung nach Anspruch 15, wobei es sich um eine organische Elektrolumineszenzvorrichtung handelt, **dadurch gekennzeichnet, dass** die Verbindung nach einem oder mehreren der Ansprüche 1 bis 10 als Matrixmaterial für phosphoreszierende Emitter in einer emittierenden Schicht eingesetzt wird.

## Claims

1. Compound of the formula (1), 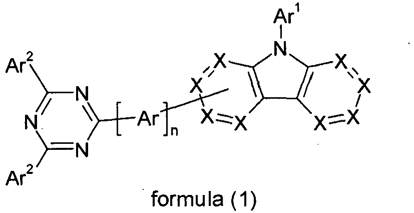 where the following applies to the symbols and indices used:
X is on each occurrence, identically or differently, CR¹ or N; or X is C if the group Ar is bonded at this position;
Ar is on each occurrence, identically or differently, a phenylene group, which may be substituted by one or more radicals R;
Ar¹ is an aromatic ring system having 6 to 24 aromatic ring atoms which contains no condensed aryl groups having more than 10 aromatic ring atoms and which may be substituted by one or more radicals R¹, or is a dibenzofuran or dibenzothiophene group, each of which may be substituted by one or more radicals R¹;
Ar² is on each occurrence, identically or differently, an aryl group, a biaryl group, a triaryl group or quateraryl group, where each individual one of the aryl groups in the above-mentioned groups has 6 to 10 aromatic ring atoms and may be substituted by one or more radicals R¹, or is a dibenzofuran or dibenzothiophene group, each of which may be substituted by one or more radicals R¹;
R is on each occurrence, identically or differently, H, D, F, CN, an aryl group, a biaryl group, a triaryl group or quateraryl group, where each individual one of the aryl groups in the above-mentioned groups has 6 to 10 aromatic ring atoms and may be substituted by one or more radicals R¹, or a carbazole group, which is linked via a carbon atom and which may also be substituted by one or more radicals R¹;
R¹ is on each occurence, identically or differently, H, D, F, CN, a straight-chain alkyl or alkoxy group having 1 to 20 C atoms or a branched or cyclic alkyl or alkoxy group having 3 to 20 C atoms, where one or more non-adjacent CH₂ groups may be replaced by R²C=CR², C≡C or O and where one or more H atoms may be replaced by D or f, or an aryl group having 6 to 10 atoms, which may be substituted by one or more radicals R², or is dibenzofuran or dibenzothiophene group, each of which may be substituted by one or more radicals R², or a carbazole group, which is linked via a carbon atom and which may also be substituted by one or more radicals R², or an aralkyl group having 6 to 10 aromatic ring atoms, which may be substituted by one or more radicals R²; two adjacent substituents R¹ here, together with the atoms to which they are bonded, may also form a mono- or polycyclic, aliphatic ring system with one another;
R² is on each occurrence, identically or differently, H, D or an aliphatic hydrocarbon radical having 1 to 20 C atoms or an aryl group having 6 to 10 ring atoms;
n is 1, 2, 3 or 4, preferably 1, 2 or 3.

2. Compound according to Claim 1, **characterised in that** a maximum of one group X per ring stands for N and the remaining groups X stand, identically or differently on each occurrence, for CR¹.

3. Compound according to Claim 1 of the formula (2), 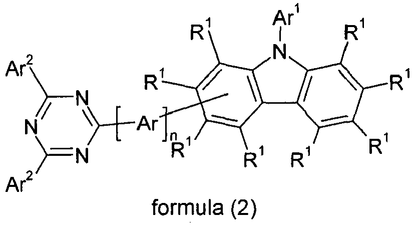 where the symbols and indices used have the meanings given in Claim 1 and the radical R¹ is not present if the group Ar is bonded at this position.

4. Compound according to one or more of Claims 1 to 3, selected from the compounds of the formulae (2a), (2b) and (2c), 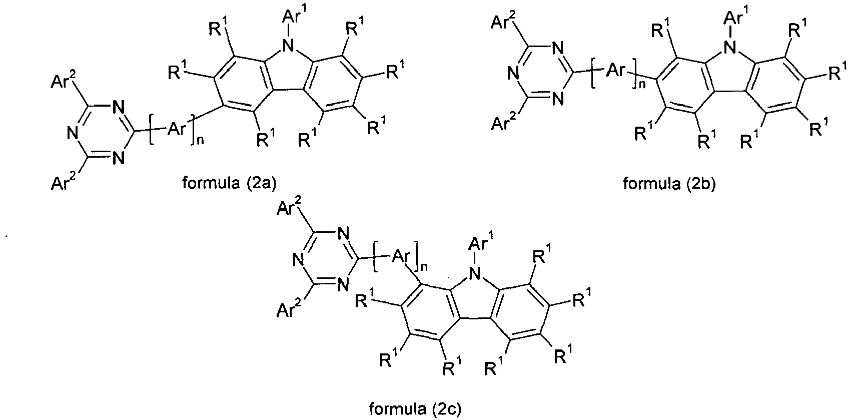 where the symbols and indices used have the meanings given in Claim 1.

5. Compound according to one or more of Claims 1 to 4, **characterised in that** n = 1, 2 or 3 and Ar is selected, identically or differently on each occurrence, from the formulae (3), (4) or (5), 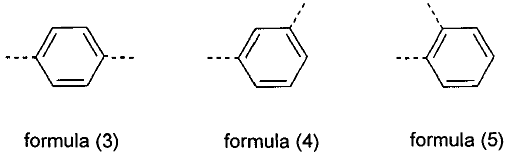 where the dashed bond in each case indicates the linking of these groups and each of these groups may be substituted by one or more radicals R.

6. Compound according to one or more of Claims 1 to 5, **characterised in that** the group -(Ar)ₙ- is selected from the formulae (6) to (18), 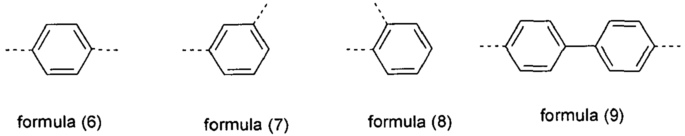 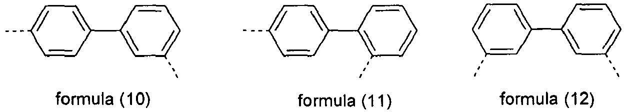 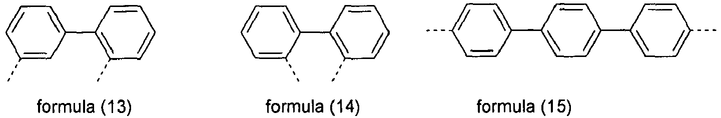 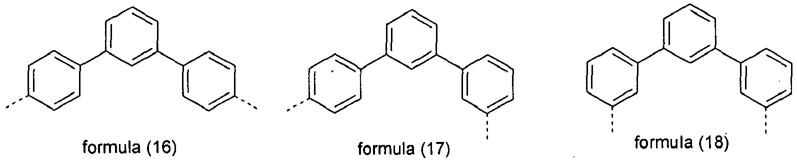 where the dashed bond in each case indicates the linking of these groups, each of these groups may also be substituted by one or more radicals R, and R and R¹ have the meanings given in Claim 1.

7. Compound according to one or more of Claims 1 to 6, **characterised in that** the radical R is selected, identically or differently on each occurrence, from the group consisting of H, D, F, CN, phenyl, biphenyl or terphenyl, where each of the aryl groups in the above-mentioned groups may be substituted by one or more radicals R¹, and R¹ stands, identically or differently on each occurrence, for H or an alkyl group having 1 to 5 C atoms; or a carbazole group, which is linked via a carbon atom and which may be substituted by one or more radicals R¹.

8. Compound according to one or more of Claims 1 to 7, **characterised in that** Ar¹ is selected from the group consisting of phenyl, biphenyl, terphenyl or quaterphenyl, each of which may be substituted by one or more radicals R¹.

9. Compound according to one or more of Claims 1 to 8, **characterised in that** Ar² is selected, identically or differently on each occurrence, from the group consisting of phenyl, biphenyl, terphenyl or quaterphenyl, each of which may be substituted by one or more radicals R¹.

10. Compound according to one or more of Claims 1 to 9, **characterised in that** the following applies to the symbols and indices:
Ar stands, identically or differently on each occurrence, for a group of the above-mentioned formula (3), (4) or (5) according to Claim 5, where n = 1, 2 or 3;
R is selected, identically or differently on each occurrence, from the group consisting of H, D, F, CN, phenyl, biphenyl or terphenyl, where each of the aryl groups may be substituted by one or more radicals R¹, and R¹ stands, identically or differently on each occurrence, for H or an alkyl group having 1 to 5 C atoms; or a carbazole group, which is linked via a carbon atom and which may also be substituted by one or more radicals R¹.
Ar¹ is selected from the group consisting of phenyl, biphenyl, terphenyl or quaterphenyl, each of which may be substituted by one or more radicals R¹;
Ar² is selected, identically or differently on each occurrence, from the group consisting of phenyl, biphenyl, terphenyl or quaterphenyl, each of which may be substituted by one or more radicals R¹;
R¹ is selected on each occurrence, identically or differently, from the group consisting of H, D, F, a straight-chain alkyl group having 1 to 10 C atoms or a branched or cyclic alkyl group having 3 to 10 C atoms, where one or more non-adjacent CH₂ groups may be replaced by R²C=CR² and where one or more H atoms may be replaced by F, or an aryl group having 6 to 10 C atoms, which may be substituted by one or more radicals R², or is a dibenzofuran or dibenzothiophene group, each of which may be substituted by one or more radicals R², or a carbazole group, which is linked via a carbon atom and which may also be substituted by one or more radicals R².

11. Process for the preparation of a compound according to one or more of Claims 1 to 10, **characterised in that** the unit (Ar²)₂triazine-(Ar)ₙ is introduced by a Suzuki coupling.

12. Mixture comprising at least one compound according to one or more of Claims 1 to 10 and at least one further compound, in particular a fluorescent or phosphorescent dopant.

13. Formulation, in particular a solution, a suspension or a miniemulsion, comprising at least one compound according to one or more of Claims 1 to 10 or a mixture according to Claim 12 and one or more solvents.

14. Use of a compound according to one or more of Claims 1 to 10 or a mixture according to Claim 12 in an electronic device, in particular in an organic electroluminescent device.

15. Electronic device comprising at least one compound according to one or more of Claims 1 to 10 or at least one mixture according to Claim 12, preferably selected from the group consisting of organic electroluminescent devices, organic integrated circuits, organic field-effect transistors, organic thin-film transistors, organic light-emitting transistors, organic solar cells, organic dye-sensitised solar cells, organic optical detectors, organic photoreceptors, organic field-quench devices, light-emitting electrochemical cells, organic laser diodes and "organic plasmon emitting devices".

16. Electronic device according to Claim 15, which is an organic electroluminescent device, **characterised in that** the compound according to one or more of Claims 1 to 10 is employed as matrix material for phosphorescent emitters in an emitting layer.

## Revendications

1. Composé de la formule (1) : 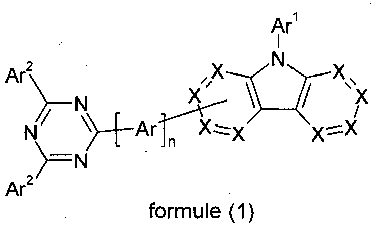 dans laquelle ce qui suit s'applique aux symboles et indices utilisés :
X est pour chaque occurrence, de manière identique ou différente, CR¹ ou N ; ou X est C si le groupe Ar est lié au niveau de cette position ;
Ar est pour chaque occurrence, de manière identique ou différente, un groupe phénylène, lequel peut être substitué par un radical ou plusieurs radicaux R ;
Ar¹ est un système de cycle aromatique comportant 6 à 24 atomes de cycle aromatique, lequel ne contient pas de groupes aryle condensés comportant plus de 10 atomes de cycle aromatique et lequel peut être substitué par un radical ou plusieurs radicaux R¹, ou est un groupe dibenzofurane ou dibenzothiophène, dont chacun peut être substitué par un radical ou plusieurs radicaux R¹ ;
Ar² est pour chaque occurrence, de manière identique ou différente, un groupe aryle, un groupe biaryle, un groupe triaryle ou un groupe quateraryle, où chaque groupe individuel des groupes aryle dans les groupes mentionnés ci avant comporte 6 à 10 atomes de cycle aromatique et peut être substitué par un radical ou plusieurs radicaux R¹, ou est un groupe dibenzofurane ou dibenzothiophène, dont chacun peut être substitué par un radical ou plusieurs radicaux R¹ ;
R est pour chaque occurrence, de manière identique ou différente, H, D, F, CN, un groupe aryle, un groupe biaryle, un groupe triaryle ou un groupe quateraryle, où chaque groupe individuel des groupes aryle dans les groupes mentionnés ci avant comporte 6 à 10 atomes de cycle aromatique et peut être substitué par un radical ou plusieurs radicaux R¹, ou un groupe carbazole, lequel est lié via un atome de carbone et lequel peut également être substitué par un radical ou plusieurs radicaux R¹ ;
R¹ est pour chaque occurrence, de manière identique ou différente, H, D, F, CN, un groupe alkyle ou alcoxy en chaîne droite comportant 1 à 20 atome(s) de C ou un groupe alkyle ou alcoxy ramifié ou cyclique comportant 3 à 20 atomes de C, où un ou plusieurs groupe(s) CH₂ non adjacents peut/peuvent être remplacé(s) par R²C=CR², C≡C ou O et où un ou plusieurs atome(s) de H peut/ peuvent être remplacé(s) par D ou F, ou un groupe aryle comportant 6 à 10 atomes de C, lequel peut être substitué par un radical ou plusieurs radicaux R², ou est un groupe dibenzofurane ou dibenzothiophène, dont chacun peut être substitué par un radical ou plusieurs radicaux R², ou un groupe carbazole, lequel est lié via un atome de carbone et lequel peut également être substitué par un radical ou plusieurs radicaux R², ou un groupe aralkyle comportant 6 à 10 atomes de cycle aromatique, lequel peut être substitué par un radical ou plusieurs radicaux R²; deux substituants R¹ adjacents ici, en association avec les atomes auxquels ils sont liés, peuvent également former un système de cycle aliphatique monocyclique ou polycyclique l'un avec l'autre ;
R² est pour chaque occurrence, de manière identique ou différente, H, D ou un radical hydrocarbone aliphatique comportant 1 à 20 atome(s) de C ou un groupe aryle comportant 6 à 10 atomes de cycle ;
n est 1, 2, 3 ou 4, de façon préférable, 1, 2 ou 3.

2. Composé selon la revendication 1, **caractérisé en ce qu'**un maximum d'un groupe X par cycle représente N et les groupes X restants représentent, de manière identique ou différente pour chaque occurrence, CR¹.

3. Composé selon la revendication 1 de la formule (2) : 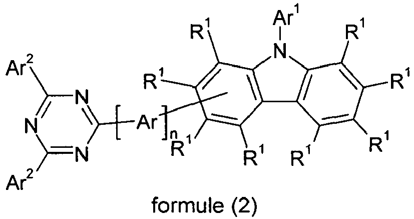 dans laquelle les symboles et indices utilisés présentent les significations données selon la revendication 1 et le radical R¹ n'est pas présent si le groupe Ar est lié au niveau de cette position.

4. Composé selon une ou plusieurs des revendications 1 à 3, choisi parmi les composés des formules (2a), (2b) et (2c) : 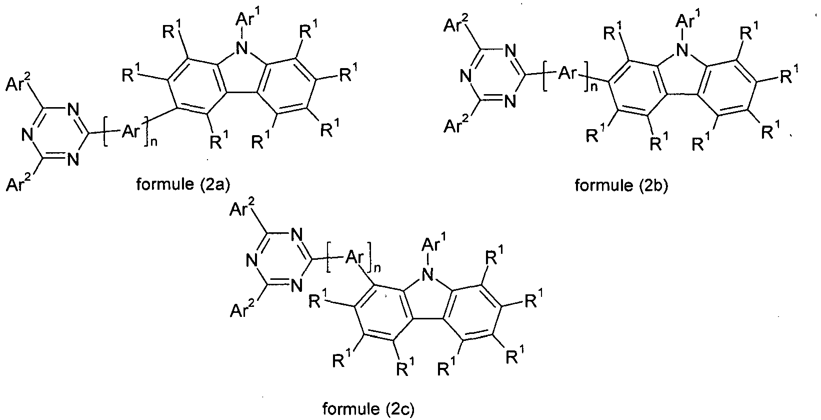 dans lesquelles les symboles et indices utilisés présentent les significations données selon la revendication 1.

5. Composé selon une ou plusieurs des revendications 1 à 4, **caractérisé en ce que** n = 1, 2 ou 3 et Ar est choisi, de manière identique ou différente pour chaque occurrence, parmi les formules (3), (4) ou (5) : 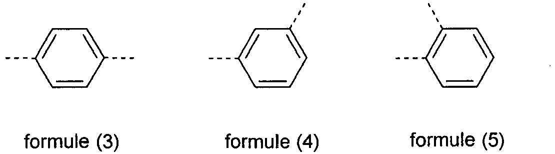 dans lesquelles le lien en pointillés, dans chaque cas, indique la liaison de ces groupes et chacun de ces groupes peut être substitué par un radical ou plusieurs radicaux R.

6. Composé selon une ou plusieurs des revendications 1 à 5, **caractérisé en ce que** le groupe -(Ar)ₙ- est choisi parmi les formules (6) à (18) : 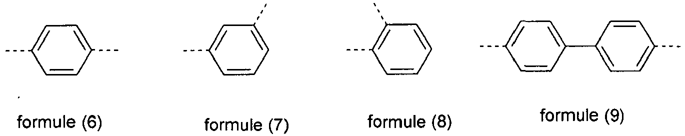 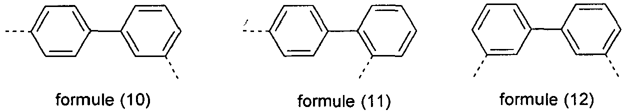 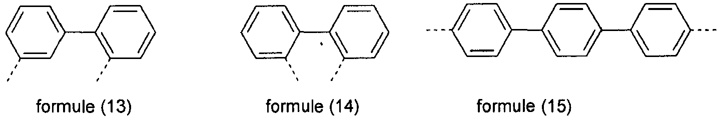 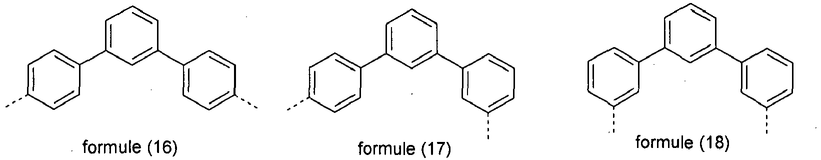 dans lesquelles le lien en pointillés, dans chaque cas, indique la liaison de ces groupes, chacun de ces groupes peut également être substitué par un radical ou plusieurs radicaux R, et R et R¹ présentent les significations données selon la revendication 1.

7. Composé selon une ou plusieurs des revendications 1 à 6, **caractérisé en ce que** le radical R est choisi, de manière identique ou différente pour chaque occurrence, parmi le groupe constitué par H, D, F, CN, phényle, biphényle ou terphényle, où chacun des groupes aryle dans les groupes mentionnés ci avant peut être substitué par un radical ou plusieurs radicaux R¹, et R¹ représente, de manière identique ou différente pour chaque occurrence, H ou un groupe alkyle comportant 1 à 5 atome(s) de C ; ou un groupe carbazole, lequel est lié via un atome de carbone et lequel peut être substitué par un radical ou plusieurs radicaux R¹.

8. Composé selon une ou plusieurs des revendications 1 à 7, **caractérisé en ce que** Ar¹ est choisi parmi le groupe constitué par phényle, biphényle, terphényle ou quaterphényle, dont chacun peut être substitué par un radical ou plusieurs radicaux R¹.

9. Composé selon une ou plusieurs des revendications 1 à 8, **caractérisé en ce que** Ar² est choisi, de manière identique ou différente pour chaque occurrence, parmi le groupe constitué par phényle, biphényle, terphényle ou quaterphényle, dont chacun peut être substitué par un radical ou plusieurs radicaux R¹.

10. Composé selon une ou plusieurs des revendications 1 à 9, caractérisé en ce qui suit s'applique aux symboles et indices :
Ar représente, de manière identique ou différente pour chaque occurrence, un groupe des formule (3), (4) ou (5) mentionnées ci avant selon la revendication 5, où n = 1, 2 ou 3 ;
R est choisi, de manière identique ou différente pour chaque occurrence, parmi le groupe constitué par H, D, F, CN, phényle, biphényle ou terphényle, où chacun des groupes aryle peut être substitué par un radical ou plusieurs radicaux R¹, et R¹ représente, de manière identique ou différente pour chaque occurrence, H ou un groupe alkyle comportant 1 à 5 atome(s) de C ; ou un groupe carbazole, lequel est lié via un atome de carbone et lequel peut également être substitué par un radical ou plusieurs radicaux R¹ ;
Ar¹ est choisi parmi le groupe constitué par phényle, biphényle, terphényle ou quaterphényle, dont chacun peut être substitué par un radical ou plusieurs radicaux R¹ ;
Ar² est choisi, de manière identique ou différente pour chaque occurrence, parmi le groupe constitué par phényle, biphényle, terphényle ou quaterphényle, dont chacun peut être substitué par un radical ou plusieurs radicaux R¹ ;
R¹ est choisi pour chaque occurrence, de manière identique ou différente, parmi le groupe constitué par H, D, F, un groupe alkyle en chaîne droite comportant 1 à 10 atome(s) de C ou un groupe alkyle ramifié ou cyclique comportant 3 à 10 atomes de C, où un ou plusieurs groupe(s) CH₂ non adjacents peut/peuvent être remplacé(s) par R²C=CR² et où un ou plusieurs atome(s) de H peut/ peuvent être remplacé(s) par F, ou un groupe aryle comportant 6 à 10 atomes de C, lequel peut être substitué par un radical ou plusieurs radicaux R², ou est un groupe dibenzofurane ou dibenzothiophène, dont chacun peut être substitué par un radical ou plusieurs radicaux R², ou un groupe carbazole, lequel est lié via un atome de carbone et lequel peut également être substitué par un radical ou plusieurs radicaux R².

11. Procédé pour la préparation d'un composé selon une ou plusieurs des revendications 1 à 10, **caractérisé en ce que** l'unité (Ar²)₂triazine-(Ar)ₙ est introduite au moyen d'un couplage de Suzuki.

12. Mélange comprenant au moins un composé selon une ou plusieurs des revendications 1 à 10 et au moins un autre composé, en particulier un dopant fluorescent ou phosphorescent.

13. Formulation, en particulier une solution, une suspension ou une mini-émulsion, comprenant au moins un composé selon une ou plusieurs des revendications 1 à 10 ou un mélange selon la revendication 12 et un ou plusieurs solvant(s).

14. Utilisation d'un composé selon une ou plusieurs des revendications 1 à 10 ou d'un mélange selon la revendication 12 dans un dispositif électronique, en particulier dans un dispositif électroluminescent organique.

15. Dispositif électronique comprenant au moins un composé selon une ou plusieurs des revendications 1 à 10 ou au moins un mélange selon la revendication 12, de façon préférable choisi parmi le groupe constitué par les dispositifs électroluminescents organiques, les circuits intégrés organiques, les transistors à effet de champ organiques, les transistors à film mince organiques, les transistors à émission de lumière organiques, les cellules solaires organiques, les cellules solaires sensibilisées par colorant(s) organiques, les détecteurs optiques organiques, les photorécepteurs organiques, les dispositifs à extinction de champ organiques, les cellules électrochimiques à émission de lumière, les diodes laser organiques et les "dispositifs à émission de plasmons organiques".

16. Dispositif électronique selon la revendication 15, lequel est un dispositif électroluminescent organique, **caractérisé en ce que** le composé selon une ou plusieurs des revendications 1 à 10 est utilisé en tant que matériau de matrice pour des émetteurs phosphorescents dans une couche d'émission.
